# EUROPEAN PATENT SPECIFICATION

(11) **EP 4 262 793 B1**
(45) Date of publication and mention of the grant of the patent: **05.02.2025**
(21) Application number: 21835776.2
(22) Date of filing: 16.12.2021
(51) Int. Cl.: A61K 31/4184, A61K 35/76, A61K 31/439, A61P 35/00, C12N 7/00

(54) **PHARMACEUTICAL COMBINATION PRODUCT COMPRISING A PROTOPARVOVIRUS AND AN ANTIVIRAL BENZIMIDAZOLE AND USES THEREOF FOR TREATING CANCER**
PHARMAZEUTISCHES KOMBINATIONSPRODUKT, DAS EIN PROTOPARVOVIRUS UND EIN ANTIVIRALES BENZIMIDAZOL ENTHÄLT, UND VERWENDUNGEN DAVON ZUR BEHANDLUNG VON KREBS
PRODUIT PHARMACEUTIQUE COMBINÉ COMPRENANT UN PROTOPARVOVIRUS ET UN BENZIMIDAZOLE ANTIVIRAL ET SES UTILISATIONS POUR LE TRAITEMENT DE CANCER

(30) Priority: 21.12.2020 EP 20216171
(43) Date of publication of application: 25.10.2023
(73) Proprietor: Marchini, Antonio, 2440 Geel (BE); Luxembourg Institute of Health (LIH), 1445 Strassen (LU)
(72) Inventor: MARCHINI, Antonio, 69120 Heidelberg (DE); PALISSOT, Valérie, 57100 Thionville-Elange (FR); MARTTILA, Tiina, 76185 Karlsruhe (DE); DORE, Gian Mario, 1424 Luxembourg City (LU)
(74) Representative: Ullrich & Naumann PartG mbB
(86) International application number: PCT/EP2021/086283
(87) International publication number: WO 2022/136125

(56) References cited:
- MARCHINI ANTONIO ET AL: "Immune Conversion of Tumor Microenvironment by Oncolytic Viruses: The Protoparvovirus H-1PV Case Study", vol. 10, 1 August 2019 (2019-08-01), pages 1848, XP055803151, Retrieved from the Internet <URL:https://www.frontiersin.org/articles/10.3389/fimmu.2019.01848/full> DOI: 10.3389/fimmu.2019.01848
- BRETSCHER CLEMENS ET AL: "H-1 Parvovirus as a Cancer-Killing Agent: Past, Present, and Future", VIRUSES, vol. 11, no. 6, 18 June 2019 (2019-06-18), pages 562, XP055803161, DOI: 10.3390/v11060562
- OCHI HIRONORI ET AL: "Direct-acting antivirals improve survival and recurrence rates after treatment of hepatocellular carcinoma within the Milan criteria", JOURNAL OF GASTROENTEROLOGY, 5 December 2020 (2020-12-05), Japan, pages 90 - 100, XP055803115, Retrieved from the Internet <URL:https://link.springer.com/content/pdf/10.1007/s00535-020-01747-y.pdf> [retrieved on 20210510], DOI: 10.1007/s00535-020-01747-y

## Description

### FIELD OF THE INVENTION

The present disclosure relates to pharmaceutical products and in particular to pharmaceutical combination products. The disclosure further relates to uses of such products in medical treatment. Embodiments of the invention have been particularly developed as pharmaceutical combination products of a protoparvovirus and an antiviral benzimidazole derivative or a pharmaceutically acceptable salt of the benzimidazole derivative as defined in the claims for use in the treatment of cancer and will be described hereinafter with reference to this application.

### BACKGROUND OF THE INVENTION

Any discussion of the background art throughout the specification should in no way be considered as an admission that such art is widely known or forms part of common general knowledge in the field.

Recent advances in diagnosis and treatment of cancer (e.g. development of novel immunotherapies such as immune checkpoint blockade) have led to a significant improvement of the five-year relative cancer survival (1). However, for some tumours such as glioblastoma, lung cancer or pancreatic carcinoma effective treatments are still lacking such that novel therapeutic options are needed. One promising approach is the use of oncolytic viruses (OVs) (2-4).

OVs are self-propagating viruses that are known for their oncotoxicity, i.e. for their ability to specifically infect and replicate in cancer cells inducing their lysis, while sparing non-cancerous cells. In addition to the release of new viral particles into the tumour bed leading to further infection of tumour cells, an OV's oncotoxicity is also linked to the release of pathogen/danger-associated molecular patterns (P/DAMPs) and tumour-associated antigens (TAAs) from the infected cells. These factors, trigger the infiltration of immune cells into the tumour microenvironment (TME) and the induction of anti-cancer immune responses (5). Due to their oncotoxicity (i.e. their anti-cancer effects) no less than 40 different OVs belonging to at least nine different virus families are presently being tested in early or late phases of clinical trials against various malignancies. One critical milestone was achieved in 2015 with marketing approval was granted in the US and in Europe for an engineered oncolytic herpes simplex virus (HSV) encoding GM-CSF (talimogene laherparepvec, T-Vec, Imlygic^{™}) for use in the treatment of malignant metastatic melanoma (6). There is justified optimism in the field that other OVs may be approved for use in the treatment of other cancers in the near future (7). However, while objective responses and promising therapeutic benefits have been observed for a number of OVs, OV treatment as a monotherapy so far rarely reproduced the impressive results obtained in preclinical studies and, therefore, OVs regularly appear to be insufficient as a standalone cancer treatments because they fail to induce complete tumour regression.

As such, major efforts are directed towards improving OVs clinical outcome and include the quest to identify other anti-cancer modalities, which might be able to synergise with an OV's oncotoxicity, for use in combination with OVs such as to increase oncotoxicity without increasing undesirable side-effects. Promising results have been achieved using combinations of an OV, namely of the protoparvovirus H-1PV, with cytotoxic anti-cancer agents such as with traditional chemotherapeutic agents (described in WO 2009/083232 A1), with histone deacetylase (HDAC) inhibitors (described in WO 2011/113600 A1) or with Bcl-2 (described in WO 2015/010782).

H-1PV is a small (~25 nm in diameter), non-enveloped icosahedral particle containing a 5.1 kb long single-stranded DNA genome (8). The genomic organization of H-1PV consists of two transcriptional units under the control of two promoters, the P4 early promoter and P38 late promoter. P4 regulates the expression of the gene encoding for the non-structural (NS) proteins (NS1 and NS2) and the P38 the one encoding for the capsid (VP) proteins (VP1, VP2, VP3) (8). The virus multiplies preferentially in fast dividing cancer cells. Without wanting to be bound by theory, this oncoselectivity is not based on a better uptake of the virus by cancerous cells, but rather is due to the fact that cancer cells overexpress factors such as cyclin A, E2F, or CREB/ATF required for virus DNA replication. Furthermore, cancer cells are often defective in their ability to mount an efficient antiviral immune response favouring viral multiplication (9). The virus is known to activate multiple cell death pathways.

Depending on cell type and growing conditions, H-1PV may induce apoptosis (10-14), necrosis (15), or cathepsin B-dependent cell death (16). The virus was able to induce oncolysis even in cancer cells resistant to TRAIL (Tumour Necrosis Factor Related Apoptosis Inducing Ligand), cisplatin and even when Bcl-2 was overexpressed (16).

In the context of cancer therapy, it has long been established that some cancer cells evade the cytotoxic effects of anti-cancer agents by being resistant or by acquiring resistance to such agents. For example, cancers resistant and/or refractory to the treatment with traditional chemotherapeutic agents such as cisplatin have long been described (16). Similarly, cancers resistant to cytotoxic HDAC and Bcl-2 inhibitors have also been described. (17,18).

Therefore, there is a need in the art to reconsider the rationale underlying new OV combination products such as to provide novel combination products based on an OV and a secondary compound with improved activity against the cancer. In particular, there is a need in the art for pharmaceutical combination products comprising an OV and a secondary compound, which enhance the OV's initial oncotoxicity but where the secondary compound itself is not an anti-cancer agent known to be possibly ineffective due to pre-existing or acquired resistance of the targeted cancer cell.

It is an object of the present invention to overcome or ameliorate at least one of the disadvantages of the prior art, or to provide a useful alternative. In particular, it is an object of the present invention to provide pharmaceutical combination products for improved protoparvovirus-based therapy.

### SUMMARY OF THE INVENTION

The references to methods of treatment in this description are to be interpreted as references to the compounds, pharmaceutical compositions and medicaments of the present invention for use in a method for treatment of the human (or animal) body by therapy.

The present invention provides a solution to the above-formulated problem based on the inventors' surprising finding that certain benzimidazole derivatives of ***Formula I,*** known for their anti-viral activity, enhance the oncotoxic effects of a protoparvovirus.

Accordingly, in a first aspect, the present invention relates to a pharmaceutical combination product comprising:
(a) a protoparvovirus, wherein said protoparvovirus is H-1 (H-1PV) or a related rodent protoparvovirus selected from Lulll, Mouse minute virus (MMV), Mouse protoparvovirus (MPV), Rat minute virus (RMV), Rat protoparvovirus (RPV) or Rat virus (RV); and
(b) an antiviral benzimidazole derivative of ***Formula I*:** 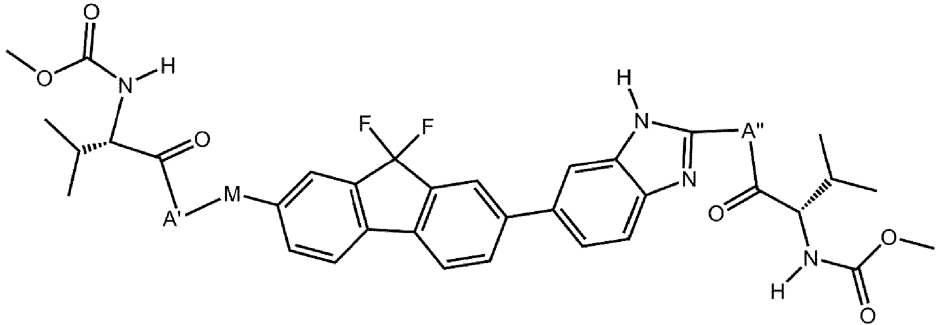
   or a pharmaceutically acceptable salt thereof,
   wherein, in the antiviral benzimidazole derivative of ***Formula I:***
      M is a 5-membered heteroaryl ring; and
      A' and A" are each independently selected from any one of (A1) to (A17): 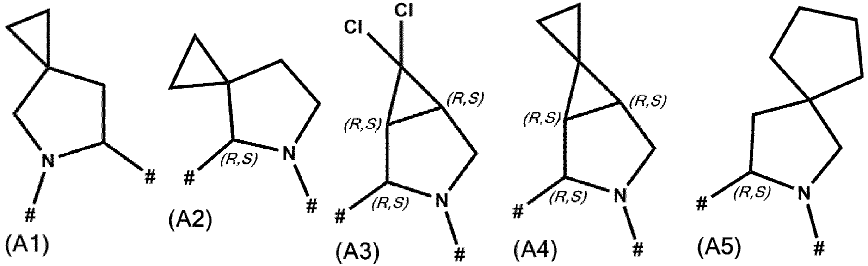 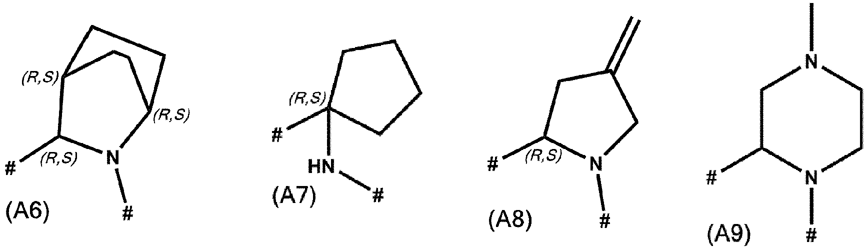 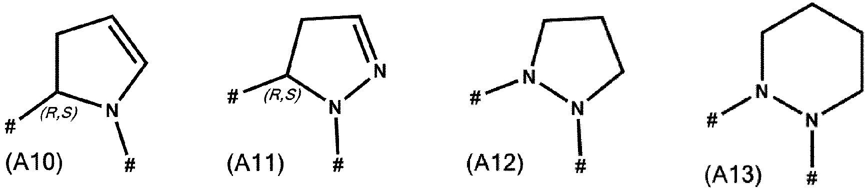 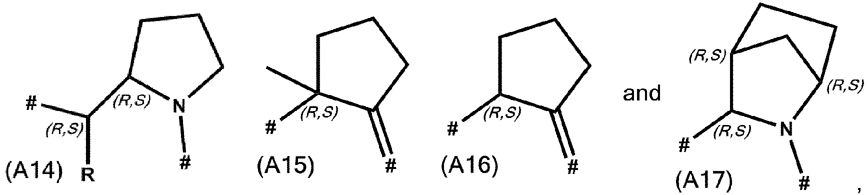
      wherein R is hydrogen or methyl.

The symbol "**#**"" shown in the respective structures of A' and A" does not indicate an atom but rather the junction of the indicated bond to an atom of the benzimidazole derivative of ***Formula I.***

Further, in instances where A' and/or A" comprise one or more carbon atoms forming chiral centres within the respective substituents, A' and/or A" will conform in their stereochemical orientation according to the constraints due to steric hindrance within the structure of the benzimidazole derivative of ***Formula I*** as well as due to the constraints due to steric hindrance within the structure of each of (A1) to (A17) themselves.

Preferably: (A2) is: 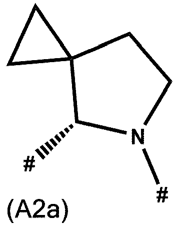 (A3) is: 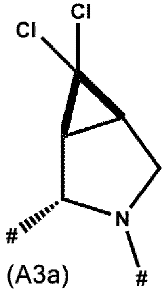 (A4) is: 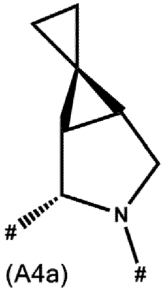 (A5) is: 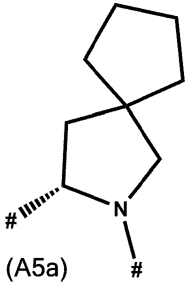 (A6) is: 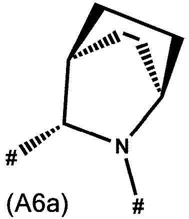 (A7) is: 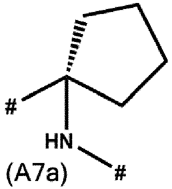 (A8) is: 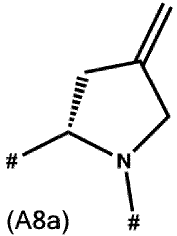 (A10) is: 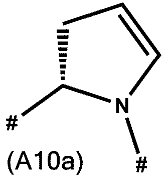 (A11) is: 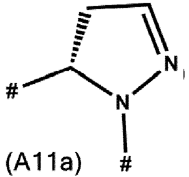 (A14) is: 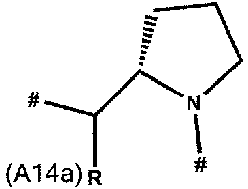 (A15) is 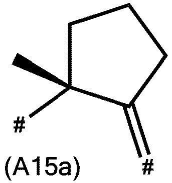 (A16) is: 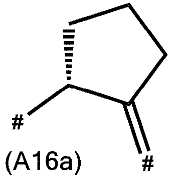 and (A17) is:

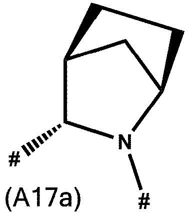

In particular embodiments of this first aspect, the antiviral benzimidazole derivative of ***Formula I*** is the antiviral benzimidazole derivative of ***Formula Ia*:** 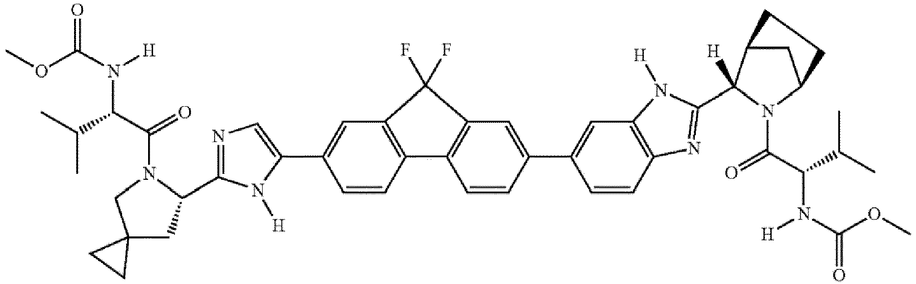 methyl *N*-[(2*S*)-1-[(6*S*)-6-[5-[9,9-difluoro-7-[2-[(1*R*,3*S*,4*S*)-2-[(2*S*)-2-(methoxycarbonylamino)-3-methylbutanoyl]-2-azabicyclo[2.2.1]heptan-3-yl]-3*H-*benzimidazol-5-yl]fluoren-2-yl]-1*H-*imidazol-2-yl]-5-azaspiro[2.4]heptan-5-yl]-3-methyl-1-oxobutan-2-yl]carbamate,
or a pharmaceutically salt thereof, and/or the protoparvovirus is H-1PV.

In particular embodiments of this first aspect, the antiviral benzimidazole derivative of ***Formula I*** is the 1:1 acetone solvate of ***Formula Ib*:** 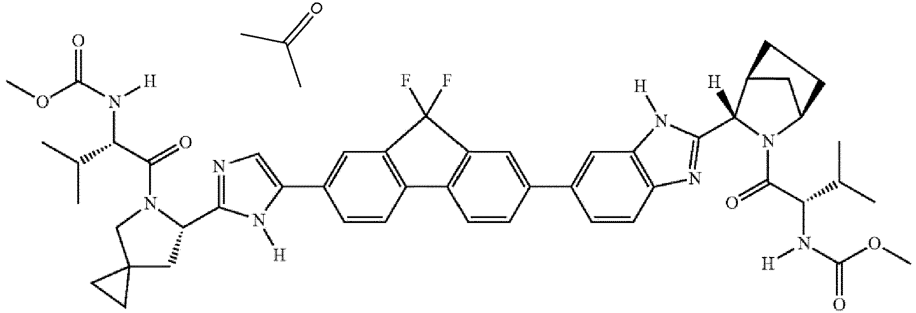 methyl *N*-[(2*S*)-1-[(6*S*)-6-[5-[9,9-difluoro-7-[2-[(1*R*,3*S*,4*S*)-2-[(2*S*)-2-(methoxycarbonylamino)-3-methylbutanoyl]-2-azabicyclo[2.2.1]heptan-3-yl]-3*H-*benzimidazol-5-yl]fluoren-2-yl]-1*H-*imidazol-2-yl]-5-azaspiro[2.4]heptan-5-yl]-3-methyl-1-oxobutan-2-yl]carbamate; propan-2-one (1:1).

Importantly, the pharmaceutical combination product according to the first aspect is particularly suitable for use in medical treatment of a patient, such as cancer treatment and, in particular, in the treatment of solid tumours (such as brain cancer, pancreatic cancer, renal cancer, lung cancer or ovarian cancer) or in the treatment of cancer-initiating precursor cells.

As such, in further aspects, the present invention also relates to a method of treatment, said method comprising administering
(a) a protoparvovirus, wherein said protoparvovirus is H-1 (H-1PV) or a related rodent protoparvovirus selected from Lulll, Mouse minute virus (MMV), Mouse protoparvovirus (MPV), Rat minute virus (RMV), Rat protoparvovirus (RPV) or Rat virus (RV); and
(b) an antiviral benzimidazole derivative of ***Formula I*** or a pharmaceutically acceptable salt thereof, as described above for the first aspect.

Similarly, in a further aspect, the present disclosure also relates to use of
(a) a protoparvovirus, wherein said protoparvovirus is H-1 (H-1PV) or a related rodent protoparvovirus selected from Lulll, Mouse minute virus (MMV), Mouse protoparvovirus (MPV), Rat minute virus (RMV), Rat protoparvovirus (RPV) or Rat virus (RV); and
(b) an antiviral benzimidazole derivative of ***Formula I*** or a pharmaceutically acceptable salt thereof, as described above for the first aspect,

in the manufacture of a pharmaceutical combination product for medical treatment such as for the treatment of cancer.

In all of the above aspects, when used in cancer treatment, the pharmaceutical combination product increases a cancer's susceptibility to protoparvovirus oncotoxicity compared to said cancer's susceptibility to an equivalent dose of protoparvovirus alone. Importantly, this susceptibility is even increased, when the pharmaceutical combination product of the invention is used in the treatment otherwise of a cancer resistant or refractory to protoparvovirus oncotoxicity.

### FIGURES

Embodiments of the invention are described, by way of example only, with reference to the accompanying drawings in which:
- Fig. 1: in accordance with the aspects of the invention and as described in more detail in Example 1 below, shows the process workflow of the experiments performed in Example 1.
- Fig. 2: in accordance with the aspects of the invention and as described in more detail in Example 1 below, shows that an FDA-approved antiviral benzimidazole derivative of ***Formula Ia*** (ledipasvir; LDV) enhances H-1PV oncolytic activity in Glioma derived SNB-19 and U-373 cell lines.
- Fig. 3.: in accordance with the aspects of the invention and as described in more detail in Example 2 below, shows the morphological effects seen in three different cell lines exposed to combination treatment with H-1PV and LDV.
- Fig. 4.: in accordance with the aspects of the invention and as described in more detail in Example 3 below, shows that low doses of LDV potentiate H-1PV oncotoxicity.
- Fig. 5.: in accordance with the aspects of the invention and as described in more detail in Example 4 below, shows that LDV enhances H-1PV oncotoxicity.
- Fig. 6.: in accordance with the aspects of the invention and as described in more detail in Example 5 below, shows that LDV synergizes with H-1PV in killing cells of the glioma-derived cell line U-373.
- Fig. 7.: in accordance with the aspects of the invention and as described in more detail in Example 6 below, shows that LDV synergizes with H-1PV in killing cancer cell lines from different solid tumours.
- Fig. 8.: in accordance with the aspects of the invention and as described in more detail in Example 7 below, shows that exposure to combination treatment with H-1PV and LDV affects cell viability at the greater extension than single-agent treatment.

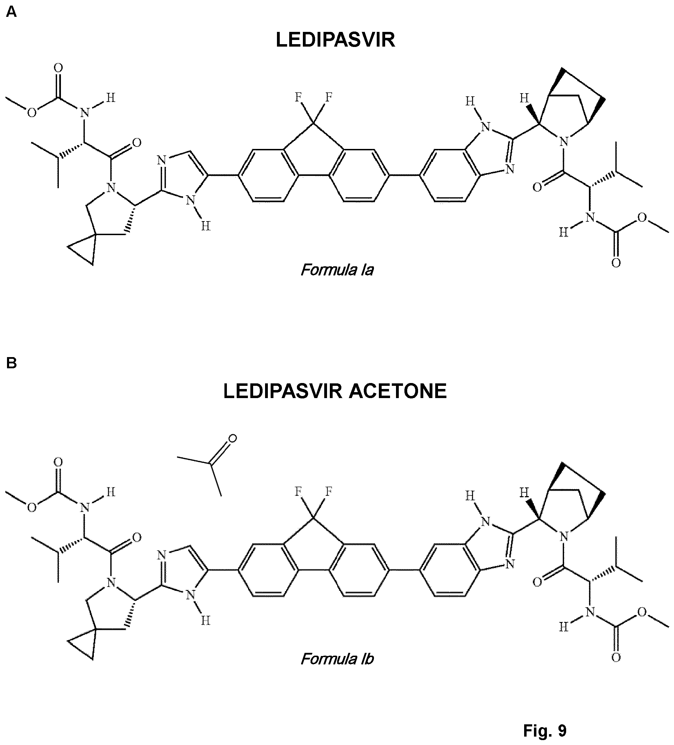

- Fig. 9.: shows the chemical formula of (A) an antiviral benzimidazole derivative of ***Formula Ia*** and (B) of an acetone solvate thereof of ***Formula Ib,*** namely of LDV and LDV acetone, respectively.

### DETAILED DESCRIPTION OF THE INVENTION

In order to provide a clear and consistent understanding of the specification and claims, and the scope to be given such terms, the following definitions are provided.

### Definitions

The term "**pharmaceutical combination product**" in the context of this disclosure includes a product in which a single composition comprises both active ingredients (a) a protoparvovirus and (b) an antiviral benzimidazole derivative. However, the term also includes a product, such as a kit, in which the two active ingredients (a) protoparvovirus and (b) antiviral benzimidazole derivative are provided in separate compositions, possibly even for simultaneous or sequential administration via the same or a different route of administration. For example, a pharmaceutical combination product in the context of the present disclosure may comprise a formulation of (a) the protoparvovirus adapted for intratumoural administration (such as a liquid for intratumoural injection), and a formulation of (b) the antiviral benzimidazole derivative adapted for oral administration (such as a tablet).

An antiviral benzimidazole derivative in accordance with the present disclosure may be converted into a pharmaceutically acceptable salt by methods well known in the art. In the context of the present invention, the term a "**pharmaceutically acceptable salt**" includes acid addition salts, formed with inorganic acids such as hydrochloric acid, hydro bromic acid, sulphuric acid, nitric acid, phosphoric acid, and the like; or formed with organic acids such as glycolic acid, pyruvic acid, lactic acid, malonic acid, malic acid, inaleic acid, fumaric acid, tartaric acid, citric acid, 3-(4-hydroxybenzoyl)benzoic acid, cinnamic acid, mandelic acid, methane sulfonic acid, ethanesulfonic acid, 1,2-ethane-disulfonic acid, 2-hydroxyethanesulfonic acid, benzenesulfonic acid, 4- chiorobenzenesulfonic acid, 2-naphthalenesulfonic acid, 4-toluenesulfonic acid, camphorsulfonic acid, lauryl sulphuric acid, gluconic acid, glutamic acid, salicylic acid, muconic acid, and the like. The term "**pharmaceutically acceptable salt**" further includes basic addition salts formed with the conjugate bases of any one of the above-listed inorganic acids, wherein the conjugate bases comprise a cationic component selected from Na⁺, K⁺, Mg²⁺, Ca²⁺, and NHgR'_{4-g}⁺; in which R' is a C₁-₃ alkyl and g is a number selected from among 0, 1, 2, 3, or 4. It should be understood that all references to a "**pharmaceutically acceptable salt**" also include solvent addition forms (solvates) or crystal forms (polymorphs) of the respective acid addition salt.

In the context of the present disclosure, the term "**medical treatment of a patient**" includes any medical treatment of a human or a non-human mammalian patient such as, without limitation, horses, cattle, dogs and cats. Expressly, the term includes treatment of liquid or solid tumours as well as of disseminated metastases and treatment targeting cancer-initiating precursor cells. The term "**liquid tumours**" here expressly includes haematological cancers such as lymphomas and leukaemias, in particular Burkitt-lymphoma, diffuse large B-cell lymphoma, T-cell acute lymphoblastic leukaemia and cutaneous T-cell lymphoma. The term "**solid tumours**" here expressly includes: solid tumours of brain cancers (e.g. glioblastoma), pancreatic cancers (e.g. pancreatic ductal adenocarcinoma), renal cancers (e.g. renal cell carcinoma), lung cancers (e.g. small cell lung cancer (SCLC) and non-small cell lung cancer (NSCLC)), breast cancers (e.g. adenocarcinomas of the breast), cervical cancers (e.g. squamous cell carcinomas of the cervix), colorectal cancers (e.g. adenocarcinomas of the colon), skin cancers (e.g. melanoma); bone cancers (e.g. osteosarcoma) and ovarian cancers (e.g. epithelial ovarian carcinomas).

In the context of the present disclosure, the term "**protoparvovirus oncotoxicity**" includes oncolytic as well as oncosuppressive activities of protoparvovirus. These activities are - typically but without limitation - consequences of "**protoparvovirus oncotropism**", i.e. through the virus' selectivity for or preferential infection of cancer cells ("**oncoselectivity**"), which leads to effective virus distribution in the tumour bed (even after intranasal of systemic virus entry), while sparing normal cells or healthy tissues. H-1PV's intrinsic oncotropism and oncoselectivity are complex phenomena based on multiple molecular determinants, which are underrepresented in normal cells, but characteristic of tumour cells, e.g. less stringent control of DNA replication, the dysregulation of signalling pathways and the impaired innate antiviral immune response in cancer cells. Protoparvovirus oncotoxicity in the context of this application also includes indirect effects such as immunogenic responses against the cancer cells triggered by protoparvovirus (including oncolytic effects on cells of a targeted tumour, which are not infected by the virus), presumably mediated through the protoparvovirus' ability to reshape the tumour micro environment.

In addition to the above definitions, and unless the context clearly requires otherwise, throughout the description and the claims, the words "**comprise**", "**comprising**", and the like are to be construed in an inclusive sense as opposed to an exclusive or exhaustive sense; that is to say, in the sense of "including, but not limited to".

Further, reference throughout this specification to "**one embodiment**", "**some embodiments**" or "**an embodiment**" means that a particular feature, structure or characteristic described in connection with the embodiment is included in at least one embodiment of the present invention. Thus, appearances of the phrases "in one embodiment", "in some embodiments" or "in an embodiment" in various places throughout this specification are not necessarily all referring to the same embodiment, but may.

As used herein, unless otherwise specified the use of the ordinal adjectives **"first", "second", "third",** etc., to describe a common object, merely indicate that different instances of like objects are being referred to, and are not intended to imply that the objects so described must be in a given sequence, either temporally, spatially, in ranking, or in any other manner.

As used herein, the term "**exemplary**" is used in the sense of providing examples, as opposed to indicating quality. That is, an "**exemplary embodiment**" is an embodiment 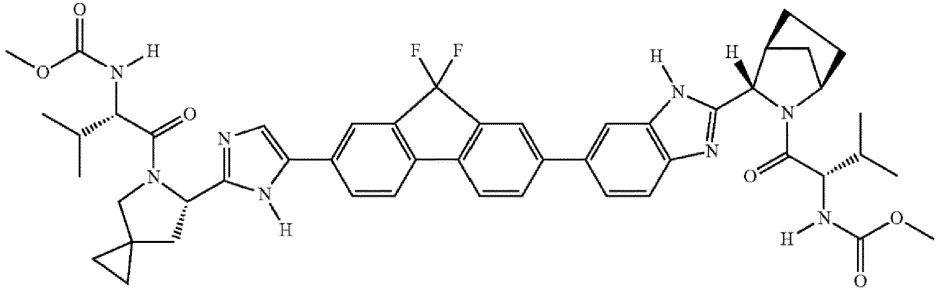 provided as an example, as opposed to necessarily being an embodiment of exemplary quality.

### Embodiments of the invention

As indicated above, the present invention is based on the inventors' surprising finding that certain benzimidazole derivatives of ***Formula I,*** anti-viral agents, enhances the oncotoxic effects of a protoparvovirus.

As such, the invention relates to a pharmaceutical combination product comprising:
(a) a protoparvovirus, wherein said protoparvovirus is H-1 (H-1PV) or a related rodent protoparvovirus selected from Lulll, Mouse minute virus (MMV), Mouse protoparvovirus (MPV), Rat minute virus (RMV), Rat protoparvovirus (RPV) or Rat virus (RV); and
(b) an antiviral benzimidazole derivative of ***Formula I*** (as described above under the heading *"SUMMARY OF THE INVENTION"* with respect to the first aspect) or a pharmaceutically acceptable salt thereof.

In particular embodiments of this first aspect, the antiviral benzimidazole derivative of ***Formula I*** is the antiviral benzimidazole derivative of ***Formula Ia*:**
methyl *N-*[(2*S*)-1-[(6*S*)-6-[5-[9,9-difluoro-7-[2-[(1*R*,3*S*,4*S*)-2-[(2*S*)-2-(methoxycarbonylamino)-3-methylbutanoyl]-2-azabicyclo[2.2.1]heptan-3-yl]-3*H*-benzimidazol-5-yl]fluoren-2-yl]-1*H*-imidazol-2-yl]-5-azaspiro[2.4]heptan-5-yl]-3-methyl-1-oxobutan-2-yl]carbamate,
or a pharmaceutically salt thereof, and/or the protoparvovirus is H-1PV.

For example, in a particular embodiments of this first aspect, the antiviral benzimidazole derivative of ***Formula I*** is the 1:1 acetone solvate of ***Formula Ib*:** 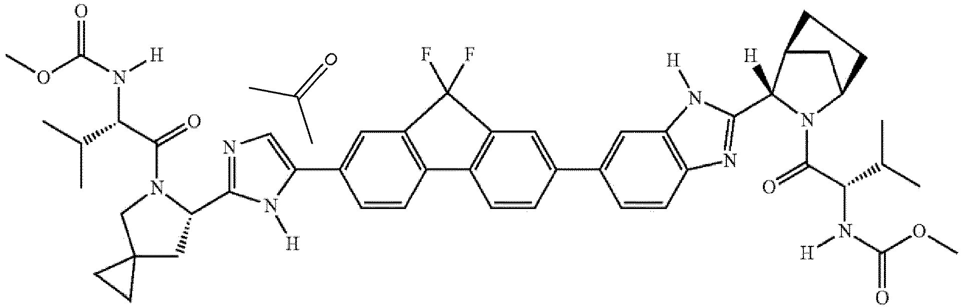 methyl *N*-[(2*S*)-1-[(6*S*)-6-[5-[9,9-difluoro-7-[2-[(1*R*,3*S*,4*S*)-2-[(2*S*)-2-(methoxycarbonylamino)-3-methylbutanoyl]-2-azabicyclo[2.2.1]heptan-3-yl]-3*H-*benzimidazol-5-yl]fluoren-2-yl]-1*H-*imidazol-2-yl]-5-azaspiro[2.4]heptan-5-yl]-3-methyl-1-oxobutan-2-yl]carbamate; propan-2-one (1:1).

H-1PV is a rat protoparvovirus presently under clinical evaluation (19). A phase I/IIa clinical study in glioblastoma patients demonstrated that H-1PV treatment is safe, well tolerated and associated with first surrogate signs of anti-cancer efficacy. These are: (i) its ability to cross the blood/tumour brain barrier after systemic administration, (ii) its good distribution in the tumour with evidence of virus replication, (iii) the induction of tumour necrosis, (iv) the immune conversion of the tumour microenvironment, and (v) the improved overall survival in comparison to historical controls (12). However, H-1PV monotherapy, as seen for other OVs, was unable to eradicate the tumours. After an initial phase in which H-1PV treatment led to tumour growth arrest and in some cases tumour regression, the tumour restarted to grow, indicating that some tumour cells escaped the treatment. As seen for many other anti-cancer agents, H-1PV resistant cancer cells may emerge, especially in the context of highly heterogeneous tumours.

H-1PV is a single-stranded DNA virus with an icosahedral capsid. Its genome contains the P4 and P38 promoters which controls the expression of NS and VP gene units, respectively. NS encodes for the non-structural proteins NS1 and NS2 while VP the capsid proteins VP1 and VP2 and the non-structural SAT protein12. In addition, to be non-pathogenic to humans, H-1PV is endowed with natural oncotoxicity, i.e. with oncolytic and oncosuppressive properties against a broad range of different tumours (19,21). However, as seen in the clinical studies, also at the preclinical level it was observed that not all cancer cell lines respond equally to H-1PV oncotoxicity, wherein some cell line cultures were found to be less sensitive or even refractory to virus infection. H-1PV has been successfully combined with other anti-cancer agents such as radiotherapy, gemcitabine, HDAC inhibitors, BH3 mimetics, bevacizumab, immune checkpoint blockade, obtaining in some cases synergistic anti-cancer effects (18). However, these agents are used in cancer treatment themselves, thus, cancer resistance to these agents may have already developed such that, at the time when these agents are combined with the virus, the efficacy of co-treatment is reduced.

Thus, the present invention, namely the combination of a protoparvovirus with an anti-viral agent that enhances protoparvovirus oncotoxicity not a known anti-cancer agent, addresses this important problem in the field.

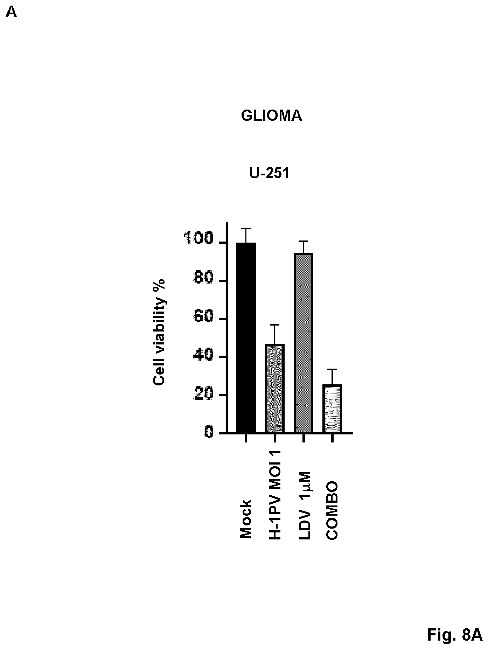

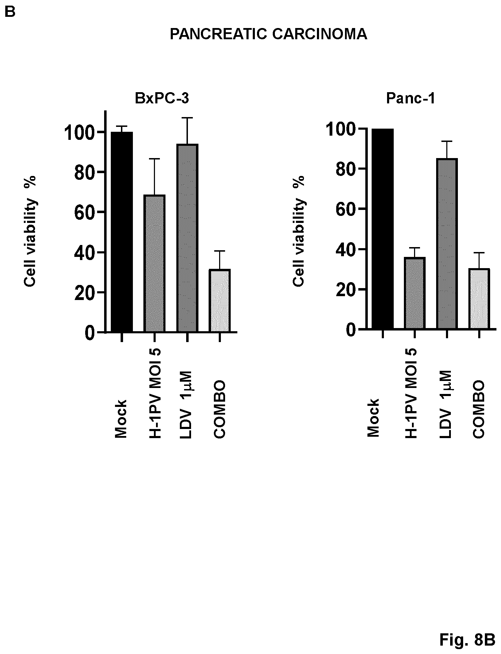

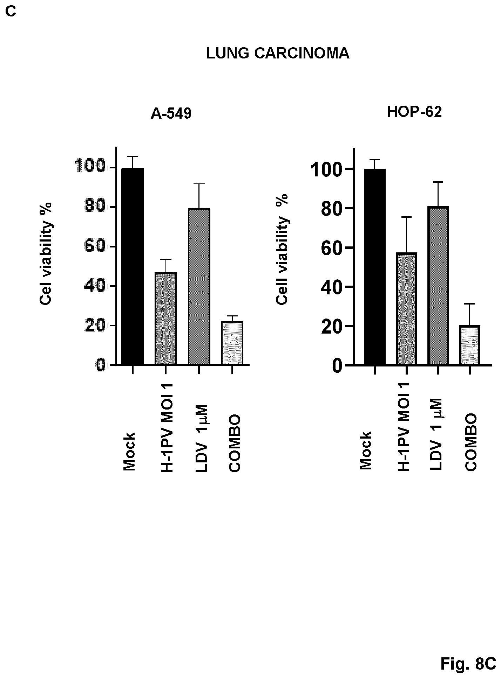

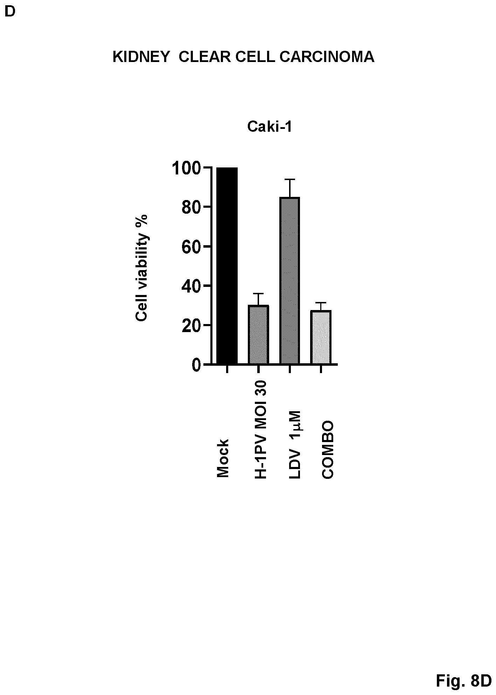

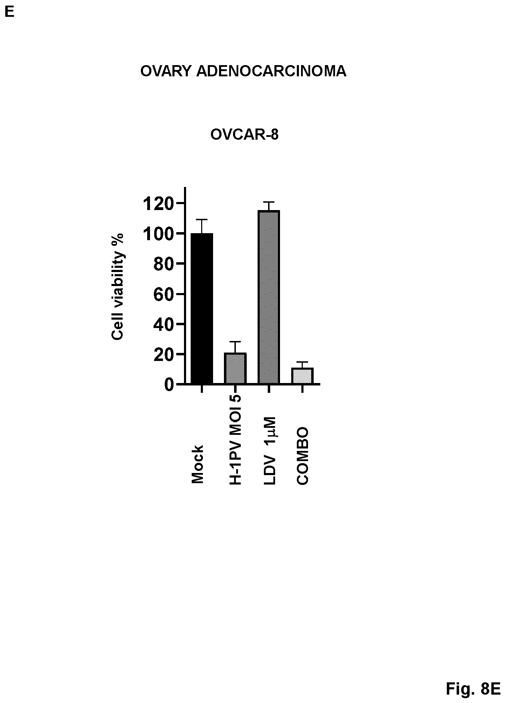

Ledipasvir (LDV; Figure 8) is a benzimidazole derivative of ***Formula Ia*** useful in the context of the present invention, but which is generally known for its clinical use in the treatment of chronic Hepatitis C, which is caused by the Hepatitis C Virus (HCV) (22). LDV is an antiviral drug, which inhibits the hyperphosphorylation of the HCV non-structural protein 5A (NS5A) thereby impairing viral RNA replication, assembly and release. LDV is orally administrated in combination with sofosbuvir under the tradename of Harvoni^{®} (Gilead Sciences). Sofosbuvir mimics the natural cellular uridine nucleotide and is incorporated by NS5A into the elongating RNA primer strand, resulting in viral chain termination (22). It is recommended as a first-line treatment against HCV genotypes 1a, 1b, 4, 5, and 6. Depending on the level of liver damage or cirrhosis, Harvoni^{®} is also used in combination with the guanosine analogue ribavirin, another antiviral agent that interferes with the synthesis of viral mRNA (23). After 12 weeks of treatment, Harvoni^{®} achieves a sustained therapeutic response in 93-99% of patients with minimal side effects (mostly limited to headache and fatigue) (24). To date, besides its role in blocking HCV (the leading cause of hepatocellular carcinoma), no direct anti-cancer activity alone or in combination with other agents have been reported for LDV.

Here, it is described - for the first time - that low doses of an antiviral benzimidazole derivative or its pharmaceutically acceptable salts as described above, potentiate H-1PV oncotoxicity in a synergistic manner.

As such, a novel, alternative use of the anti-viral benzimidazole derivative of ***Formula Ia*,** LDV, as a booster of H-1PV oncotoxicity is provided. Notably, LDV enhances the H-1PV oncolytic activity also in cancer cell lines particularly resistant to monotherapy with H-1PV.

Importantly, the pharmaceutical combination product of the present invention may comprise (a) the protoparvovirus and (b) the antiviral benzimidazole derivative as separate entities. As such, the combination product may be a kit, preferably for use in medical treatment such as cancer treatment, comprising a protoparvovirus composition and a separate antiviral benzimidazole derivative composition for simultaneous or sequential administration.

In this regard, it is noteworthy that the two separate entities of a pharmaceutical combination product of the invention (i.e. such as a kit) may be formulated for the same or for distinct routes of administration. For example, the protoparvovirus composition may be formulated for intratumoural or systemic or intranasal administration, while the antiviral benzimidazole derivative composition may be formulated for intratumoural or systemic or oral administration. In preferred embodiments, (a) the protoparvovirus is formulated for intratumoural administration and (b) the antiviral benzimidazole derivative is formulated for oral administration.

In alternative embodiments, the pharmaceutical combination product of the present invention may comprise (a) the protoparvovirus and (b) the antiviral benzimidazole derivative in a single composition. For example, both (a) and (b) may be formulated for intratumoural or systemic administration.

As explained above, the pharmaceutical combination product of the present invention is particularly suitable for use in medical treatment, especially for use in cancer treatment, of a patient. The cancer treatment in this context (and as expressly defined above) includes the treatment of solid tumours and/or the targeting of cancer-initiating precursor cells or small disseminated metastasis. In particular embodiments of the invention, the solid tumours are tumours caused by brain cancer, pancreatic cancer, renal cancer, lung cancer or ovarian cancer.

As explained above, the pharmaceutical combination of the present invention increases a cancer's susceptibility to protoparvovirus oncotoxicity compared to said cancer's susceptibility to an equivalent dose of protoparvovirus alone. Given that, at the doses used, LDV does not show any oncotoxicity or anti-cancer effect itself, it synergises with the protoparvovirus to enhance the oncotoxicity of the virus.

In some of these embodiments, the pharmaceutical combination product for use according to the invention allows for the treatment of a cancer typically resistant or refractory to protoparvovirus oncotoxicity.

### Examples

The invention is further described by the following non-limiting Examples.

### Methods and materials

### Cell lines and culture media

The human glioblastoma derived cell lines U-373 and U-251 were provided by Dr. Iris Augustin (DKFZ, Heidelberg, Germany). Pancreatic ductal adenocarcinoma (PDAC) derived cell lines AsPC-1, Panc-1 and BxPC-3 were purchased from American Type Culture Collection (ATCC) and provided by LGC standards GmbH, Wesel, Germany. The lung cancer derived A-549 and HOP-62, prostate carcinoma derived PC3 and DU-145, glioma derived SNB-19, breast cancer derived T47D, Ovarian cancer derived OVCAR-8 and clear cell renal carcinoma derived Caki-I were obtained from the National Cancer Institute (NCI, Bethesda, MD, USA). U-373, U-251 and Panc-1 cell lines were cultured in Dulbecco's modified Eagle's medium (DMEM, Sigma-Aldrich, St. Luis, MO, USA ). All other human cancer cell lines were grown in Roswell Park Memorial Institute medium 1640 (RPMI, Sigma-Aldrich, St. Luis, MO, USA). All media were supplemented with 10% heat inactivated fetal bovine serum (FBS, Sigma), 2 mM L-Glutamine (Gibco, Thermo Scientific, Waltham, USA). DMEM medium was also supplemented with 100 U/ml penicillin and 100 µg/ml streptomycin. All cells were grown at 37°C in 5% CO₂, 95% humidity and routinely checked for mycoplasma contamination.

### FDA approved drug library screening

Identification of the benzimidazole derivatives useful in the present invention was achieved through screening of panels of FDA-approved drugs (SelleckChem library: http://www.selleckchem.com/screening/fda-approved-drug-library.html; Selleck Chemicals LLC, Houston, TX, USA). The screening was performed with the Agilent BioCel automated workstation housed in an Ermaflux L2 biosafety cabinet (equipped with charcoal filters to trap gases). The glioma cell lines SNB-19 and U-373 were used for the screening. Cells were seeded with the BRAVO pipettor in 384 well cell culture microplate at 15% confluence in medium supplemented with 10% FBS and penicillin/streptomycin. After overnight incubation half of the plates was infected with wild-type H-1PV at MOI of 1 plaque forming unit (pfu)/cell, the other half kept untreated. All cells were then treated with 5 concentrations of each of the 1,443 compounds (5-point dose curve incorporating a 4-fold dilution step in duplicates, range 4 - 0.156 µM). After 72 h, mock and infected cells were then incubated with PrestoBlue^{®} cell viability reagent for 1 hour (resazurin-based; Thermofisher Scientific, Illkirch-Graffenstaden, France). PrestoBlue^{®} is a cell viability indicator by using the reducing power of living cells to quantitatively measure the proliferation of cells. The PrestoBlue^{®} reagent contains a cell-permeant compound that is blue in colour and virtually non-fluorescent. When added to cells, the PrestoBlue^{®} reagent is modified by the reducing environment of the viable cell and becomes highly fluorescent. Conversion is proportional to the number of metabolically active cells and therefore can be measured quantitatively. Quantitation of fluorescent signal intensity of the cell culture supernatant was carried out using the HTS-ready Berthold MITHRAS LB 940 fluorescent plate reader at excitation and emission wavelengths of 569/586 nm (reduced resazurin form resorufin). All screening plates were subjected to stringent quality control measures using Z-factor score analysis using the GraphPad Prism software and available R-packages.

### LDH assay

In an lactate dehydrogenase assay (**LDH assay**) LDH released from a cell, e.g. due to cell lysis, oxidizes lactate to generate NADH, which then in turn reacts with a colorimetric substrate to generate a quantifiable colour change, which is directly correlated to the amount of LDH released and can, as such, used as a measure of cell lysis.

Cancer cell lines were seeded at a density of 3,000 (U-251, BxPC-3, Panc-1, DU-145, A-549, HOP-62 and Caki-1) or 4,000 (SNB-19, AsPC-1, PC3 and U-373) cells/well in 96-well plates in 50 µl of their respective culture medium supplemented with 10% heat inactivated FBS. After 24 h, 50µl of FBS-free medium with or without H-1PV and/or Ledipasvir (LDV; Selleck Chemicals LLC, Houston, TX, USA) was added. Cells were incubated for 3-5 days and then subjected to lactase dehydrogenase LDH assay (CytoTox 96 non-radioactive cytotoxicity assay, Promega Madison, WI, USA), according to the manufacturer's instructions. For each condition tested, six replicates were prepared of which three were used for calculating the total lysis in the presence of detergent as previously described (25).

### MTT assay

For the analysis of cell viability, the metabolic activity of nicotinamide adenine dinucleotide phosphate (NADPH)-dependent cellular oxidoreductase enzymes was assessed by MTT assays, as previously described (26). These enzymes catalyses the reduction of 3-(4,5-dimethylthiazol-2-yl)-2,5-diphenyl-2H-tetrazolium bromide (MTT) to a soluble coloured formazan dye. The absorbance of the formazan dye was measured at 595 nm by using an ELISA plate reader. Cells were plated in 96 well plates at the density of 3,000 cells/well in 50 µl of their respective culture medium supplemented with 10% heat inactivated FBS. After 24 h, 50 µl of FBS-free medium with or without H-1PV and/or LDV was added. The viability of infected cells was expressed as the ratio of the corresponding absorbance to that of non-infected cells taken arbitrarily as 100%. The mean percentage of MTT activity was calculated from at least three replicates for each condition tested.

### Virus production, purification and titration

Wild type H1-PV was produced and purified as previously described (27). Virus titration was carried out by quantitative real-time PCR and plaque assay according to the methods described in (26). Viral DNA was extracted using Qiamp MiniElute Virus Spin Kit (Qiagen, Hilden, Germany) according to the manufacturer's instruction manual.

### Real-time detection of cell viability and proliferation

Human cancer cells were seeded into a 96-well E-plate (ACEA Biosciences, San Diego, CA, USA) at a density of 4000 cells/well. After 24 h, cells were infected or not with H-1PV and grown in the presence or absence of LDV. Growth of mock treated cells and virus infected cells was monitored in real-time every 30 min, for 6 days using the xCELLIgence System (ACEA). Cell proliferation was expressed as normalized cell index (CI), a parameter directly proportional to the number of attached cells per well and, therefore, strictly correlated with cell proliferation and viability. The growth curves represent the averages of the results of at least three replicates

### Example 1: FDA-approved-drugs screening reveals the anti-viral benzimidazole derivative of Formula la, LDV, as a booster of H-1PV oncotoxicity.

In order to improve H-1PV clinical outcome, it is essential to identify anti-cancer agents that can enhance its oncotoxicity. To this end, the FDA- approved drug library (SelleckChem library: http://www.selleckchem.com/screening/fda-approved-drug-library.html) was screened. The library includes 1,443 compounds belonging to different functional classes, e.g. anti-cancer drugs, COX2 inhibitors, antiinflammatories, anti-diabetics, anti-arthritics, anti-coagulants, etc., selected in order to maximize chemical and pharmaceutical diversity. Being FDA approved and already in use in the clinic, these compounds have known and well characterized bioactivities and have undergone extensive safety and bioavailability testing. The drugs were tested alone or in combination with H-1PV in SNB-19 and U- 373 glioma cells. As a positive control for the screening ABT-737 - a BH3 mimetic that inhibits pro-survival Bcl-2 family members (often overexpressed in cancer cells) - was used. ABT-737 was previously shown to enhance H-1PV oncotoxicity in a synergistic manner both *in vitro* (using different cancer cell lines of different origins) and *in vivo* (xenograft rat model of human pancreatic carcinoma) (28).

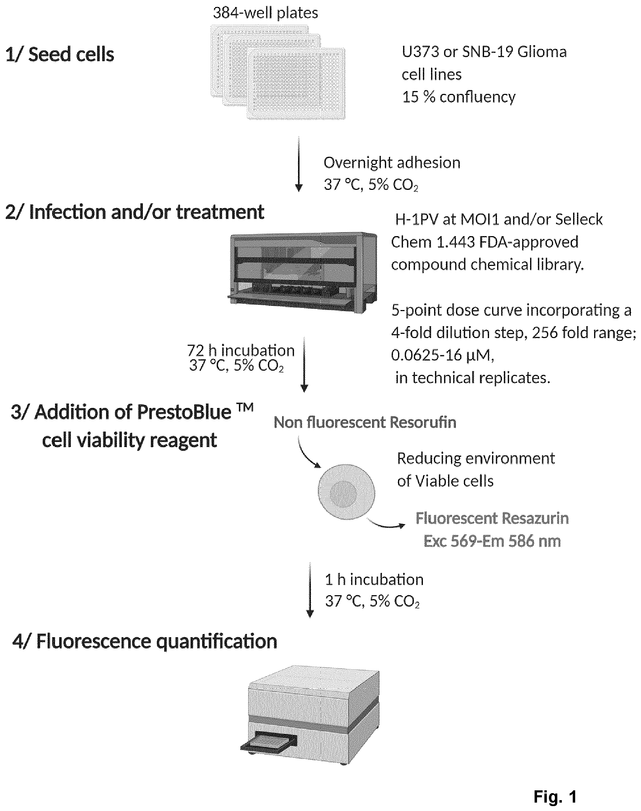

In agreement with these results, ABT-737 synergized with H-1PV in killing SNB-19 and U-373 glioma cells in the drug screening (data not shown). Among top candidates, positive results were also obtained with three other drugs (ABT-199, ABT-263 and LDV). Of note, ABT-199 and ABT-263, are also BH3 mimetics belonging to the same family of ABT-737. These results provide further evidence that BH3 mimetics synergize with H-1PV in killing cancer cells and also provide confidence on the validity of the screening procedure. In conclusion, and as shown in Figure 1, the drug screening for the first time showed that addition of LDV enhances the oncolytic activity of H-1PV (Figure 1). As the drug is used as an inhibitor of HCV, our results identify a novel and alternative use of LDV as an anti-cancer agent in combination with oncolytic H-1PV.

Specifically, Figure 1 shows the experimental set up chosen to reveal that LDV acts as a booster of H-1PV oncotoxicity. In this set-up, Glioma-derived SNB-19 and U-373 cells were plated in 384-well plates and were then treated (or not) with the FDA approved drugs in combination (or not) with H-1PV used at a MOI of 1 (pfu/cell) for 72 h. The cells were then processed for PrestoBlue^{®} cell viability assay as described in the materials and methods section.

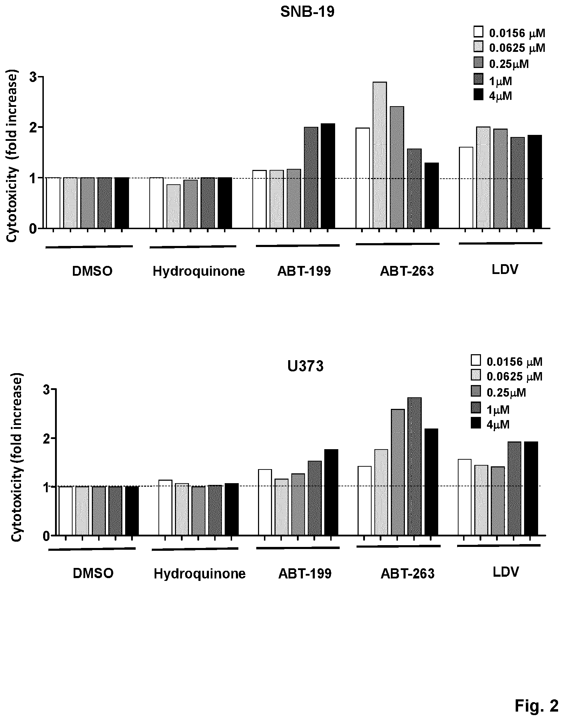

Figure 2 then illustrates selected results from the drug screen. The drug candidates achieving the highest synergistic effect with H-1PV,, were the two BH3 mimetics ABT-199 and ABT-263, and the anti-viral benzimidazole derivative of Formula la, LDV . An example of a FDA-approved-drug that did not interact with H-1PV (Hydroquinone) is also shown. The columns of the graphs of Figure 2 represent the fold increase in cytotoxicity calculated using the PrestoBlue values compared to the cytotoxicity seen for cells infected only with H-1PV (DMSO control) and normalized to the cytotoxicity seen for the various drugs at the concentrations indicated in non-infected cells (not shown). As such, the experimental results for the drugs tested, i.e. for ABT-199, ABT-263 and LDV in combination with H-1PV infection, indicate the fold increase in H-1PV cytotoxicity achieved through the respective combinations.

### Example 2: Optical microscopy analysis confirms that low doses of LDV enhance the anti-cancer activity of H-1PV.

Two glioblastoma cell lines (SNB-19 and U-373) and a pancreatic cell line (AsPC-1) displaying different susceptibility to H-1PV infection were treated (or not) with increasing amounts of H-1PV (MOI, pfu/cell) in the presence or absence of 4 µM LDV. At 4 days post-treatment, at the concentration used, LDV did not induce any evident cytotoxic effects and cells were growing similarly to untreated cells. On the contrary, evident signs of cytopathic effects and morphological changes were observed in all three H-1PV treated cancer cell lines resulting in a lower density of cells attached to the culture plates. Cytopathic effects were more marked in H-1PV/LDV co-treated cancer cell lines, showing that low doses of the drug potentiate H-1PV oncotoxicity (Figure 2).

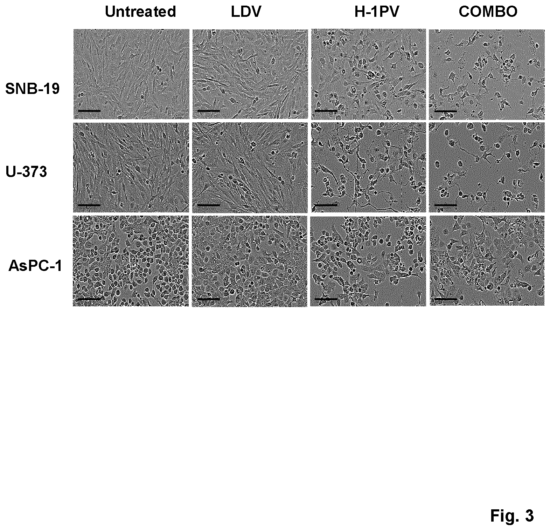

Specifically, Figure 3 shows the morphological effects seen in three different cell lines exposed to combination treatment with H-1PV and LDV. The cancer cell lines indicated in Figure 3 were plated at the same density. After 24 h, cells were infected (or not) with H-1PV used at the MOI (pfu/cell) of 1 (SNB-19), or 100 (U-373 and AsPC-1) in combination (or not) with 4 µM LDV. Light microscopy images were acquired using the IncuCyte ZOOM^{™} live cell imaging system (Essen BioScience, MI USA) at 96 h after treatment. The Figure shows representative images of the cells taken at a magnification of 10X.

### Example 3.- Real time monitoring of cell proliferation confirmed that low doses of LDV potentiate H-1PV oncotoxicity.

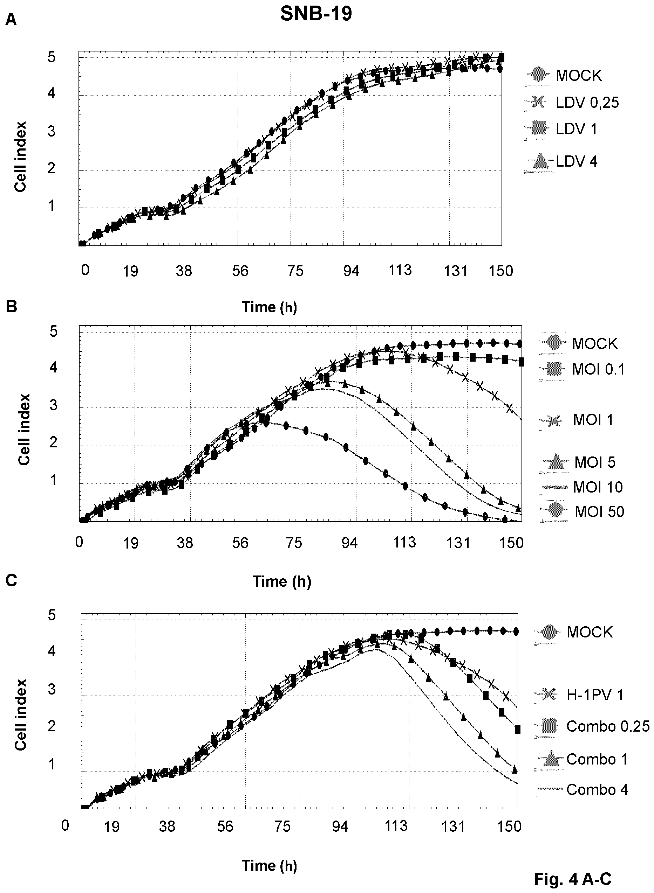

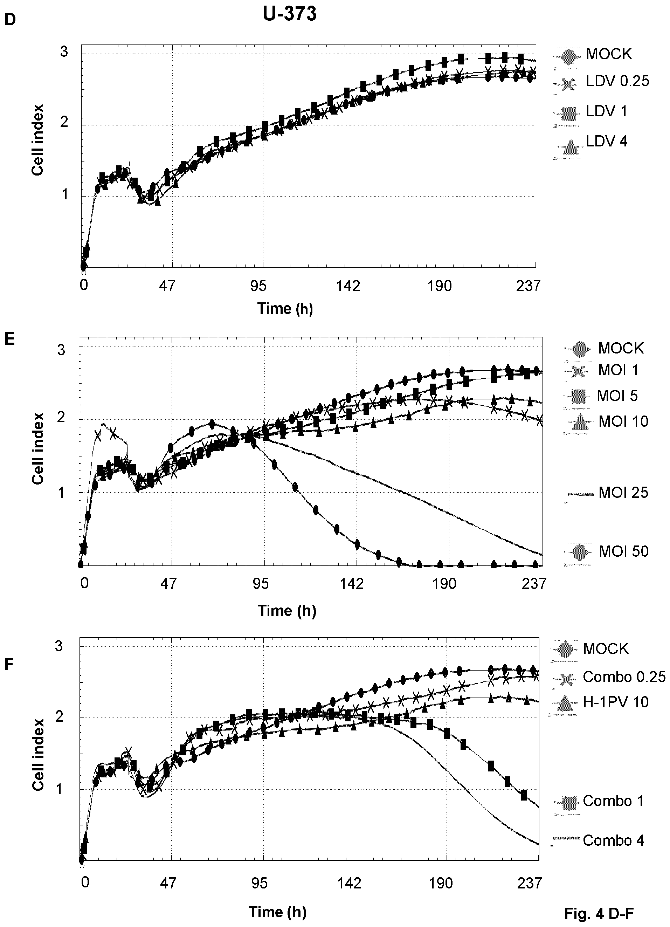

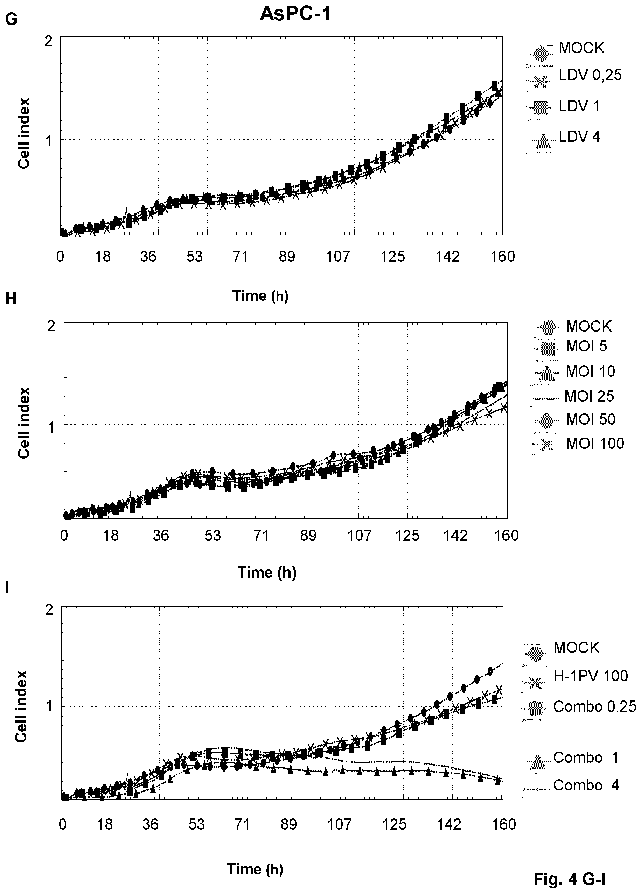

The xCELLIgence system allows the monitoring in real time of cell proliferation and viability. This system was used to confirm the anti-cancer effect of H-1PV/LDV co-treatment. Three cancer cell lines were used for these experiments, namely glioma cell lines SNB-19 and SNB-19, and pancreatic carcinoma cell line AsPC-1. Cells were treated (or not) with H-1PV in the presence or absence of LDV. Cell proliferation was monitored every 30 min for a total of 150 hours. In agreement with previous observation (Figure 3), addition of LDV - at the concentrations used - did not affect cell proliferation (Figure 4 A and G). H-1PV had a different effect on the growth and viability of the two cancer cell lines. SNB-19 cells were killed by H-1PV in a dose-dependent manner (as noted by a decrease in cell index over the time) (Figure 4B). U-373 cells were killed only when H-1PV was used at high concentrations of 25 or 50 but were resistant when the virus was used at lower doses (e.g. MOI 10) (Figure 4E). AsPC-1 cells were resistant to H-1PV even when the virus was used at high concentrations (up to a MOI of 100 pfu/cell) and cells were growing like mock treated cells (Figure 4H). Addition of LDV to the culture medium, enhanced the ability of H-1PV to stop the growth of all three cancer cell lines tested and affect their viability (Figure 4C, F and I). Notably, the addition of low doses of the drug (1 and 4 µM), made AsPC-1 cells sensitive to H-1PV oncolytic activity (Figure 4F).

Specifically, Figure 4 shows that low doses of LDV potentiate H-1PV oncotoxicity. Cancer cells were seeded in a 96-well E-plate. After 24 h, cells were treated (or not) with the indicated amount of LDV (µM) and/or H-1PV (MOls, pfu/cell). Cell proliferation was monitored in real-time using the xCELLIgence system. The values in Figure 4 are expressed as normalized cell index and plotted against time. Each experimental condition was tested at least in triplicates and average values are illustrated.

### EXAMPLE 4: Low doses of LDV enhance the oncolytic activity of H-1PV.

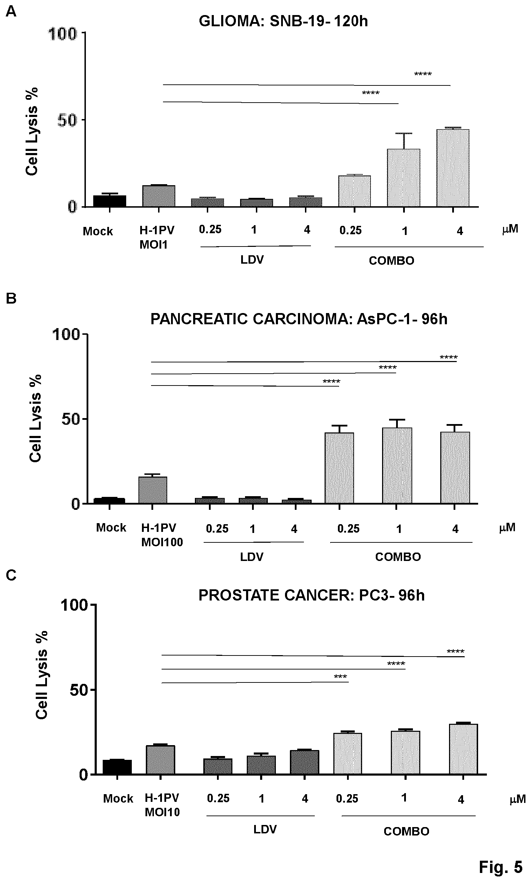

To further confirm the results shown in Examples 1-3, the oncolytic activity of H-1PV/LDV combination, was analysed by LDH assays. Human SNB-19 (glioma), AsPC-1 (pancreatic carcinoma), and PC3 (prostate cancer) cell lines were infected (or not) with H-1PV in the absence or presence of increasing concentrations of LDV. At the concentrations used, LDV alone did not induce cell lysis and H-1PV triggered cell lysis only in a small fraction of the cells. Cell lysis was much stronger in H-1PV/LDV co-treated cells, providing evidence of synergistic oncolytic activity between the two agents (Figure 5).

Specifically, Figure 4 shows that LDV enhances H-1 PV oncotoxicity. Cancer cells lines derived from glioma (SNB-19, panel A) pancreatic (AsPC-1, panel B) and prostate cancer (PC3, panel C), were grown for 24 h before to be treated as indicated for 120 (SNB19) or 96 (AsPC1 and PC3) h. Cells were then processed for LDH assay to assess treatment-induced cell lysis. Columns show average cell lysis values with standard deviation bars. For each condition tested, at least three independent experiments were performed in triplicate. Anova was used for statistical analysis of the results. **** indicates a p-value of ≤ 0.001.

### EXAMPLE 5: LDV potentiates the oncolytic activity of H-1PV.

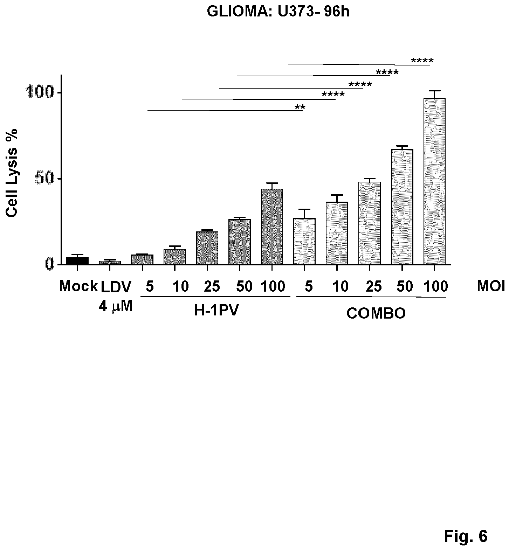

U-373 glioma cells treated (or not) with 4 µM LDV were infected (or not) with increasing viral MOls (pfu/cell) and treatment-induced cell lysis was assessed by LDH assay. Under our experimental conditions, LDV did not induce cell lysis (Figure 6). H-1PV induced cell lysis in a dose-dependent manner killing almost 50% of the cells when used at the highest MOI tested of 100 pfu/cell. A strong increase of cell lysis (two-three folds) was observed when the cells were treated with H-1PV in combination with LDV with all viral MOls tested. Total cell lysis was achieved when the virus was used with LDV at MOI of 100 confirming that the two agents cooperate in killing cancer cells (Figure 6).

Specifically, Figure 6 shows that LDV synergizes with H-1PV in killing cells of the glioma-derived cell line U-373. U373 glioma cells, were grown for 24 h before being treated for 96 h as indicated. Cells were then processed for LDH assay to measure treatment-induced cell lysis. The columns of the graphs of Figure 6 show average cell lysis values with standard deviation bars. For each condition tested, at least three independent experiments were performed in triplicate and Anova was used for statistical analysis of the results. ** indicates a p-value of ≤ 0.01; **** indicates a p-value of ≤ 0.001.

### EXAMPLE 6: LDV boosts the oncolytic activity of H-1PV against cancer cell lines derived from different solid tumours

H-1PV can target and kill a broad spectrum of cancer cell lines from different tumour entities. The positive results shown in Examples 1 to 5, led to further analysis of whether the enhancement effect of LDV on H-1 PV oncolysis can generally be obtained in other cancer cell lines displaying different susceptibility to H-1PV killing activity. To this end the H-1PV/LDV combination was further tested in additional seven cancer cell lines from different tumour entities, namely glioma (U-251), pancreatic carcinoma (BxPC-3 and Panc-1) prostate (Du-145), lung (A-549 and Hop-62) and clear cell renal carcinoma (Caki-1). Cells were treated (or not) with H-1PV, LDV or the two agents in combination. Treatment-induced cell lysis was analysed by LDH assay. In agreement with the results shown in Figures 5 and 6, treatment with LDV did not induced cell lysis. H-1PV at the MOls used was also not efficient in its oncolytic activity and higher MOls would have been required to induce stronger oncolytic activity (data not shown). However, H-1PV mediated oncolysis was enhanced by the addition of LDV in all cancer cell lines tested. Together, these results provide further evidence that H-1PV/LDV co-treatment kills cancer cells in a synergistic manner.

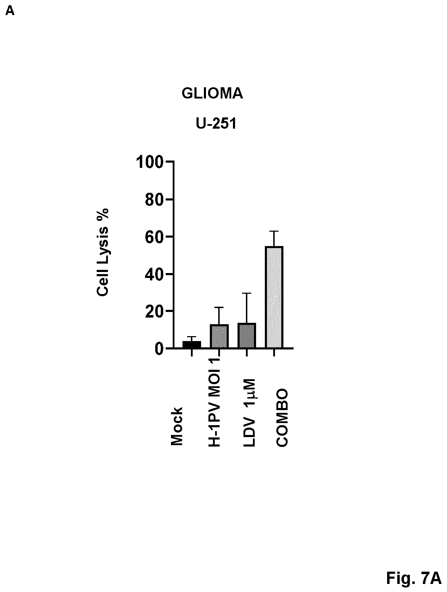

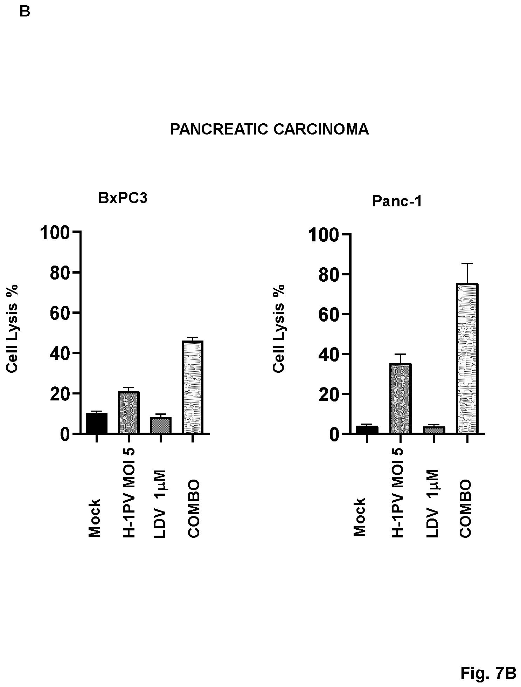

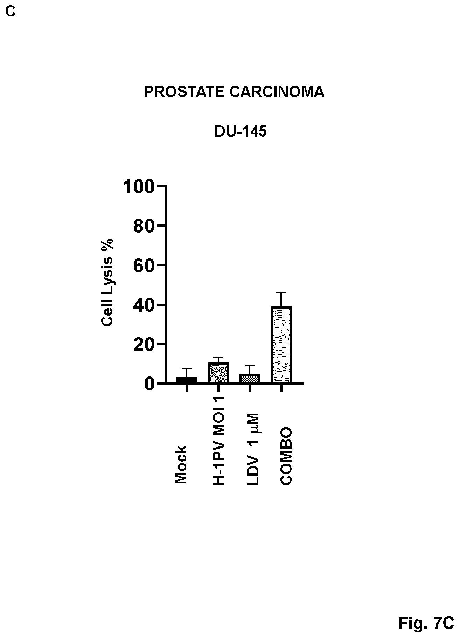

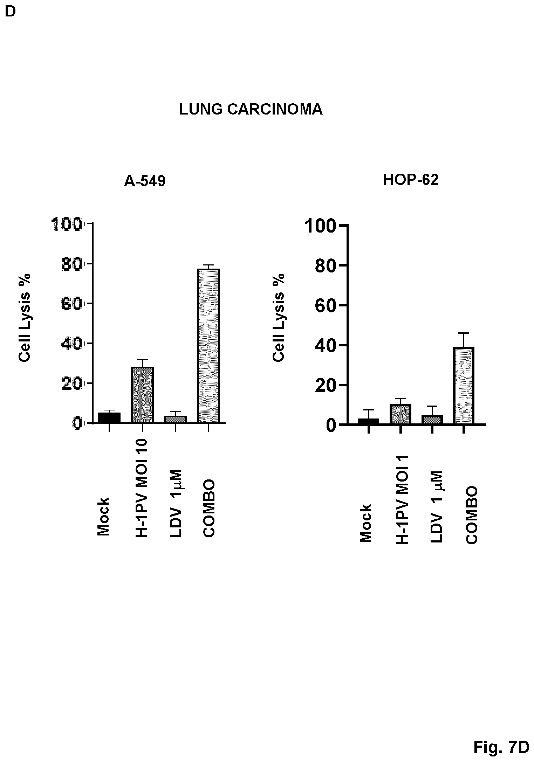

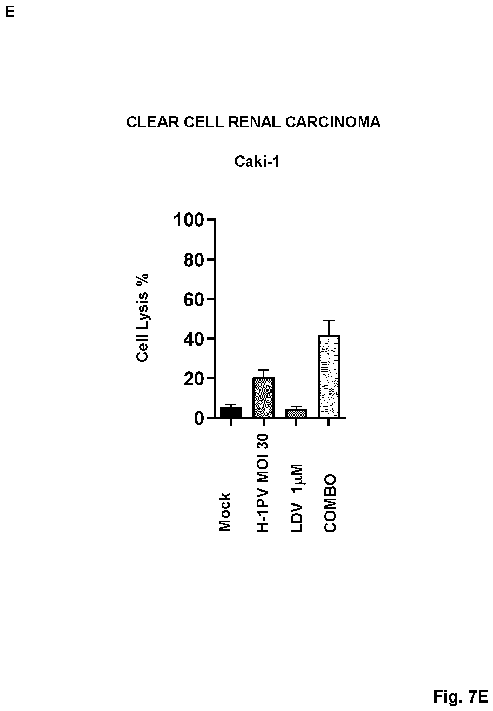

Specifically, Figure 7 shows that LDV synergizes with H-1 PV in killing cancer cell lines from different solid tumours. Cancer cells lines derived from glioma (U-251, panel A), pancreatic carcinoma (BxPC-3 and Panc-1, panel B), prostate cancer (DU-145, panel C), lung cancer (A-549 and HOP-62, panel D) or clear cell renal carcinoma (Caki-1, panel E), were grown for 24 h before to be treated (or not) with the indicated amounts of LDV and/or H-1PV at the indicated MOI (pfu/cell). After 96 h, cells were then processed for LDH assay to measure treatment-induced cell lysis. Columns show average values with standard deviation bars. Results from a typical experiment performed at least in triplicate are shown.

### EXAMPLE 7: H-1PV/LDV co-treatment affects the viability of human cancer cell lines

In order to further confirm the results shown in Examples 1-6, with an independent method, cell viability was evaluated using MTT assays. U-251 (glioma), BxPC-3 and Panc-1 (pancreatic carcinoma), A-549 and HOP-62 (lung carcinoma), Caki-1 (kidney clear cell carcinoma) and OVCAR-8 (ovary adenocarcinoma) were infected (or not) with H-1PV in the presence or absence of LDV. At 72-96 hour post-treatment, cells were subjected to MTT assays. In agreement with previous results, also MTT assays confirmed decreased viability in H-1PV/LDV co-treated cells in comparison with singly treated cells (Figure 8 and data not shown).

Specifically, Figure 8 shows that exposure to combination treatment with H-1PV and LDV affects cell viability at the greater extension than single-agent treatment. Glioma (U-251), pancreatic carcinoma (BxPC-3 and Panc-1), lung carcinoma (A-549 and HOP-62), kidney clear cell carcinoma (Caki-1) and ovary adenocarcinoma (OVCAR-8) cells were plated at a density of 4000 cells/well in a 96 well plate. After 24 h, cells were infected (or not) with H-1PV at the indicated MOls (PFU/cells) in the presence or absence of the indicated amounts of LDV. At 96 h post-treatment, cells were processed for MTT assay to determine cell viability. The columns of the graphs of Figure 8 represent average values calculated from at least three replicates with relative standard deviation bars. Results from a typical experiment are shown.

### REFERENCES

(1) Miller, K.D., et al. Cancer treatment and survivorship statistics, 2019. CA Cancer J Clin 69, 363-385 (2019).
(2) Achard, C et al. Lighting a Fire in the Tumor Microenvironment Using Oncolytic Immunotherapy. EBioMedicine 31, 17-24 (2018).
(3) Kaufman, H.L., Kohlhapp, F.J. & Zloza, A. Oncolytic viruses: a new class of immunotherapy drugs. Nat Rev Drug Discov 14, 642-662 (2015).
(4) Keller, B.A. & Bell, J.C. Oncolytic viruses-immunotherapeutics on the rise. J Mol Med (Berl) 94, 979-991 (2016).
(5) Marchini, A., Daeffler, L., Pozdeev, V.I., Angelova, A. & Rommelaere, J. Immune Conversion of Tumor Microenvironment by Oncolytic Viruses: The Protoparvovirus H-1PV Case Study. Front. Immunol. 10, 1848 (2019).
(6) Ledford, H. Cancer-fighting viruses win approval. Nature 526, 622-623 (2015).
(7) Conry, R.M., Westbrook, B., McKee, S. & Norwood, T.G. Talimogene laherparepvec: First in class oncolytic virotherapy. Hum Vaccin Immunother 14, 839-846 (2018).
(8) Marchini, A., Scott, E. M. & Rommelaere, J. Overcoming Barriers in Oncolytic Virotherapy with HDAC Inhibitors and Immune Checkpoint Blockade. Viruses 8, doi: 10.3390/v8010009 (2016).
(9) Andtbacka, R. H. et al. Talimogene Laherparepvec improves Durable Response Rate in Patients With Advanced Melanoma. J Clin Oncol 33, 2780-2788, doi: 10.1200/JCO.2014.58.3377 (2015).
(10) Foimtzilas, C., Patel, S. & Mahalingam, D. Review: Oncolytic virotherapy, updates and future directions. Oncotarget 8, 102617-102639, doi: 10.18632/oncotarget.18309 (2017).
(11) Cotmore SF, Tattersall P. Parvoviral host range and cell entry mechanisms. Adv Virus Res 70: 183-232 (2007).
(12) Nuesch JP, Lacroix J, Marchini A, Rommelaere J. Molecular pathways: rodent parvoviruses-mechanisms of oncolysis and prospects for clinical cancer treatment. Clin Cancer Res 18: 3516-3523 (2012).
(13) Hristov G, Kramer M, Li J, El-Andaloussi N, Mora R, Daeffler L, Zentgraf H, Rommelaere J, Marchini A. Through Its Nonstructural Protein NS1, Parvovirus H-1 Induces Apoptosis via Accumulation of Reactive Oxygen Species. J Virol 84: 5909-5922 (2010).
(14) Ohshima T, Iwama M, Ueno Y, Sugiyama F, Nakajima T, Fukamizu A, Yagami K. Induction of apoptosis in vitro and in vivo by H-1 parvovirus infection. The Journal of general virology 79 ( Pt 12): 3067-3071 (1998).
(15) Rayet B, Lopez-Guerrero JA, Rommelaere J, Dinsart C. Induction of programmed cell death by parvovirus H-1 in U937 cells: connection with the tumor necrosis factor alpha signalling pathway. J Virol 72: 8893-8903 (1998).
(16) Bretscher, C. & Marchini, A. H-1 Parvovirus as a Cancer-Killing Agent: Past, Present, and Future. Viruses 11 (2019).
(17) Lee JH, Choy ML, Marks PA. Mechanisms of resistance to histone deacetylase inhibitors. Adv Cancer Res. 116:39-86 (2012)
(18) Ryan CE, Davids MS. BCL-2 Inhibitors, Present and Future. Cancer J. 25(6):401-409 (2019).
(19) Geletneky, K., et al. Oncolytic H-1 Parvovirus Shows Safety and Signs of Immunogenic Activity in a First Phase I/IIa Glioblastoma Trial. Mol Ther (2017)*.*
(20) Marchini, A., Bonifati, S., Scott, E.M., Angelova, A.L. & Rommelaere, J. Oncolytic parvoviruses: from basic virology to clinical applications. Virol J 12, 6 (2015).
(21) Angelova, A. & Rommelaere, J. Immune System Stimulation by Oncolytic Rodent Protoparvoviruses. Viruses 11(2019).
(22) Scott, L.J. Ledipasvir/Sofosbuvir: A Review in Chronic Hepatitis C. Drugs 78, 245-256 (2018).
(23) Ahmed, H., et al. Safety and efficacy of sofosbuvir plus ledipasvir with and without ribavirin for chronic HCV genotype-1 infection: a systematic review and meta-analysis. Antivir Ther 22, 369-379 (2017).
(24) Stokes, W., et al. The Efficacy and Safety of 12 Weeks of Sofosbuvir and Ledipasvir versus Sofosbuvir, Ledipasvir, and Ribavirin in Patients with Chronic Hepatitis C, Genotype 1, Who Have Cirrhosis and Have Failed Prior Therapy: A Systematic Review and Meta-Analysis. Can J Gastroenterol Hepatol 2017, 6468309 (2017).
(25) El-Andaloussi, N., et al. Generation of an adenovirus-parvovirus chimera with enhanced oncolytic potential. J Virol 86, 10418-10431 (2012).
(26) Daeffler, L., Horlein, R., Rommelaere, J. & Nuesch, J.P. Modulation of minute virus of mice cytotoxic activities through site-directed mutagenesis within the NS coding region. J Virol 77, 12466-12478 (2003).
(27) Leuchs, B., Roscher, M., Muller, M., Kurschner, K. & Rommelaere, J. Standardized large-scale H-1PV production process with efficient quality and quantity monitoring. J Virol Methods 229, 48-59 (2016).
(28) Li, J., Schroeder, L., Geletneky, K., Rommelaere, J. & Marchini, A. Cancer therapy with a parvovirus combined with inhibitors of the anti-apoptotic Bcl-2 proteins. European Patent EP3024491 (2016).

## Claims

1. A pharmaceutical combination product comprising:
(a) a protoparvovirus, wherein said protoparvovirus is H-1 (H-1PV) or a related rodent protoparvovirus selected from Lulll, Mouse minute virus (MMV), Mouse protoparvovirus (MPV), Rat minute virus (RMV), Rat protoparvovirus (RPV) or Rat virus (RV); and
(b) an antiviral benzimidazole derivative of ***Formula I:*** 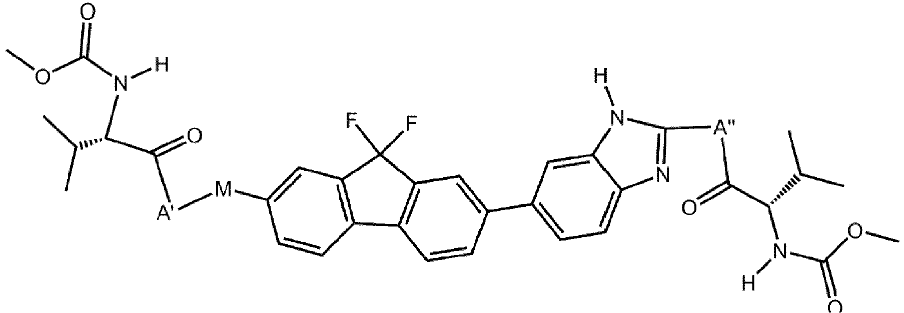 or a pharmaceutically acceptable salt thereof,
wherein, in the antiviral benzimidazole derivative of ***Formula I:***
M is a 5-membered heteroaryl ring; and
A' and A" are each independently selected from any one of (A1) to (A17): 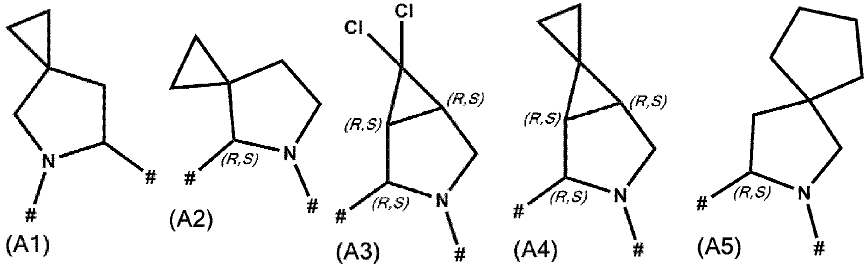 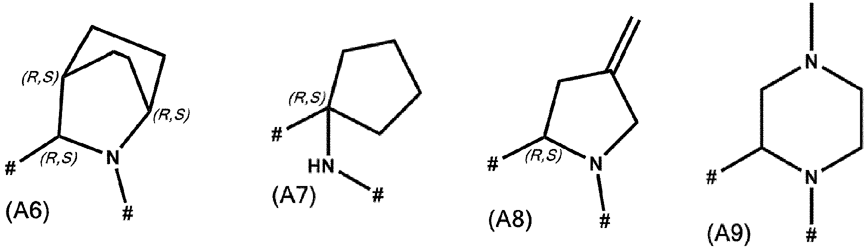 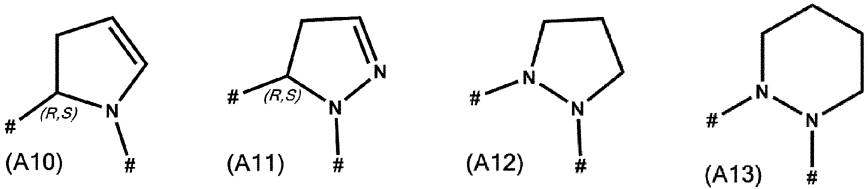 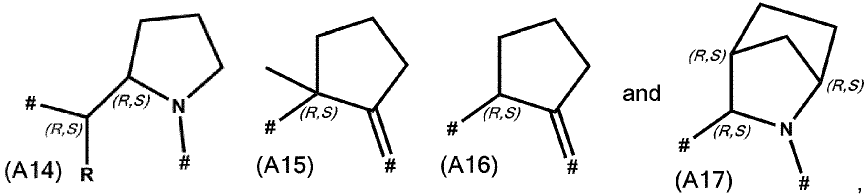 wherein R is hydrogen or methyl.

2. The pharmaceutical combination product according to claim 1, wherein (A2) is: 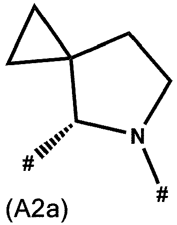 (A3) is: 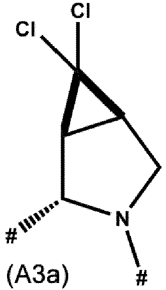 (A4) is: 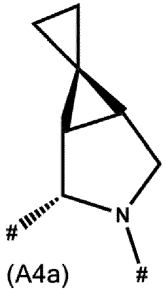 (A5) is: 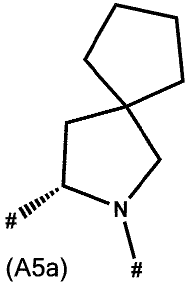 (A6) is: 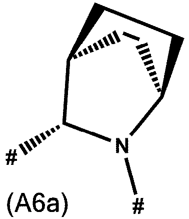 (A7) is: 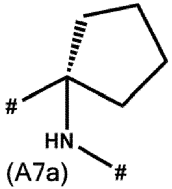 (A8) is: 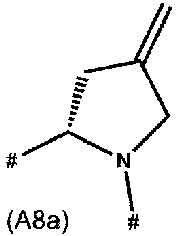 (A10) is: 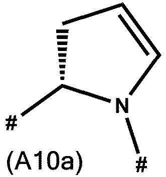 (A11) is: 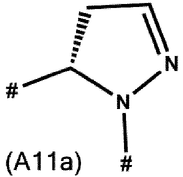 (A14) is: 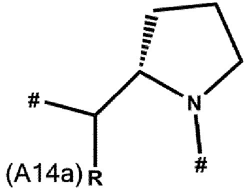 (A15) is 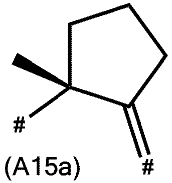 (A16) is: 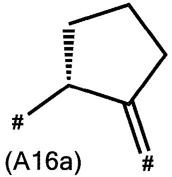 and (A17) is:

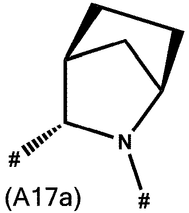

3. The pharmaceutical combination product according to claim 1, wherein the antiviral benzimidazole derivative of ***Formula I*** is the antiviral benzimidazole derivative of ***Formula Ia**:* 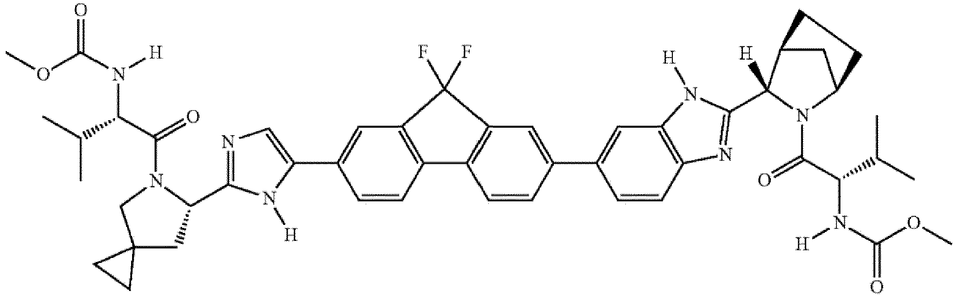
methyl *N*-[(2*S*)-1-[(6*S*)-6-[5-[9,9-difluoro-7-[2-[(1*R*,3*S*,4*S*)-2-[(2*S*)-2-(methoxycarbonylamino)-3-methylbutanoyl]-2-azabicyclo[2.2.1]heptan-3-yl]-3*H*-benzimidazol-5-yl]fluoren-2-yl]-1*H*-imidazol-2-yl]-5-azaspiro[2.4]heptan-5-yl]-3-methyl-1-oxobutan-2-yl]carbamate,
or a pharmaceutically salt thereof.

4. The pharmaceutical combination product according to any one of claims 1 to 3, wherein the protoparvovirus is H-1PV.

5. The pharmaceutical combination product according to any one of claims 1 to 4, comprising (a) the protoparvovirus and (b) the antiviral benzimidazole derivative as separate entities.

6. The pharmaceutical combination product according to any one of claims 1 to 4, comprising (a) the protoparvovirus and (b) the antiviral benzimidazole derivative in a single composition.

7. The pharmaceutical combination product according to any one of claims 1 to 6 for use in medical treatment of a patient.

8. The pharmaceutical combination product for use according to claim 7, wherein said medical treatment is cancer treatment.

9. The pharmaceutical combination product for use according to claim 7 or 8, wherein said treatment is treatment of solid tumours or cancer-initiating precursor cells.

10. The pharmaceutical combination product for use according to claim 8, wherein said cancer treatment is treatment of brain cancer, pancreatic cancer, renal cancer, lung cancer or ovarian cancer.

11. The pharmaceutical combination product for use according to any one of claims 7 to 10, wherein said treatment increases a cancer's susceptibility to protoparvovirus oncotoxicity compared to said cancer's susceptibility to an equivalent dose of protoparvovirus alone.

12. The pharmaceutical combination product for use according to any one of claims 7 to 11, wherein said treatment is treatment of a cancer resistant or refractory to protoparvovirus oncotoxicity.

13. The pharmaceutical combination product for use according to any one of claims 7 to 12, wherein said treatment comprises: intratumoural or systemic or intranasal administration of (a) the protoparvovirus; and intratumoural or systemic or oral administration of (b) the antiviral benzimidazole derivative.

14. The pharmaceutical combination product for use according to claim 13, wherein said treatment comprises: intratumoural administration of (a) the protoparvovirus; and oral administration of (b) the antiviral benzimidazole derivative

15. The pharmaceutical combination product for use according to any one of claims 7 to 14 wherein said treatment comprises administering (a) the protoparvovirus and (b) the antiviral benzimidazole derivative to said patient simultaneously or sequentially.

## Patentansprüche

1. Ein pharmazeutisches Kombinationsprodukt, umfassend:
(a) ein Protoparvovirus, wobei das Protoparvovirus H-1 (H-1PV) ist oder ein verwandtes Protoparvovirus von Nagetieren, ausgewählt aus Lulll, Maus-Minutevirus (MMV), Maus-Protoparvovirus (MPV), Ratten-Minutevirus (RMV), Ratten-Protoparvovirus (RPV) oder Rattenvirus (RV), ist; und
(b) ein antivirales Benzimidazolderivat der ***Formel I*:** 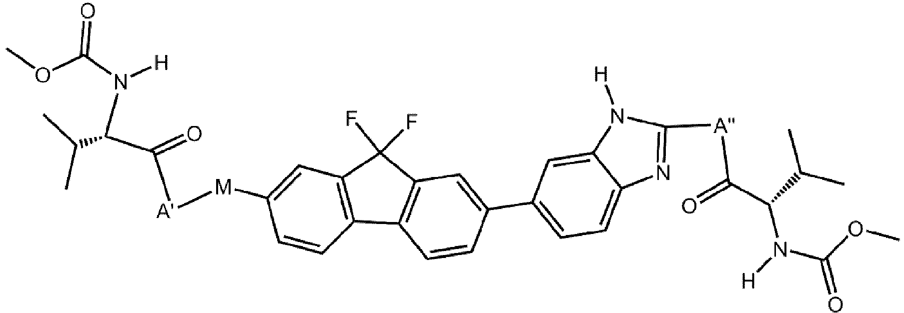 oder ein pharmazeutisch akzeptables Salz davon,
wobei in dem antiviralen Benzimidazolderivat der ***Formel I:***
M ein fünfgliedriger Heteroarylring ist; und
A' und A" jeweils unabhängig voneinander ausgewählt sind aus (A1) bis (A17): 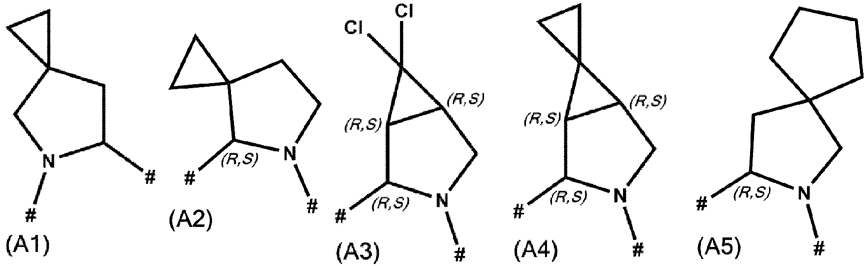 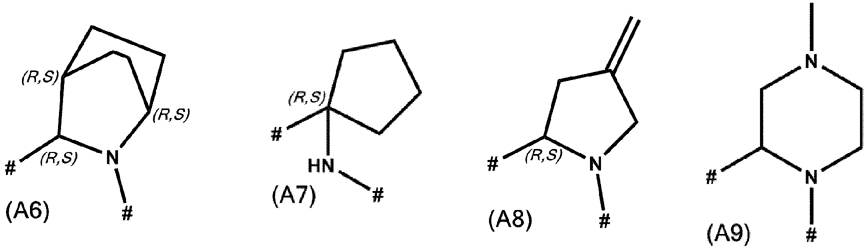 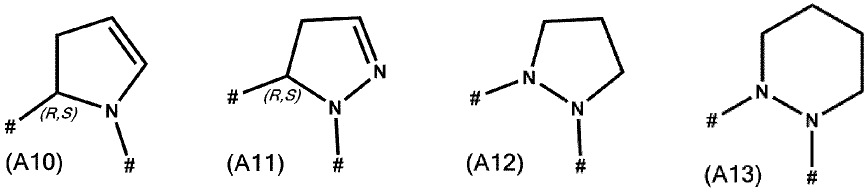 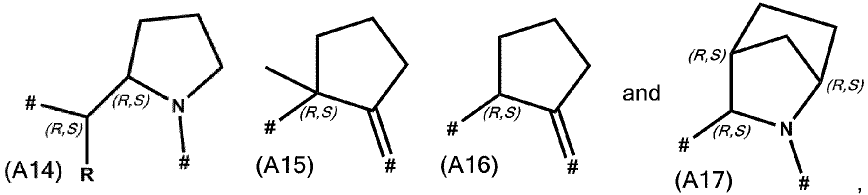 wobei R Wasserstoff oder Methyl ist.

2. Das pharmazeutische Kombinationsprodukt nach Anspruch 1, wobei (A2) ist: 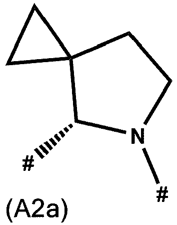 (A3) ist: 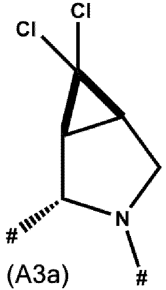 (A4) ist: 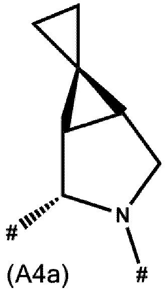 (A5) ist: 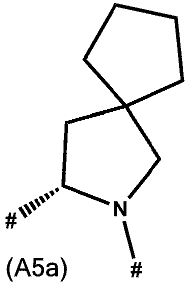 (A6) ist: 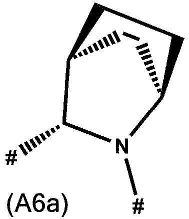 (A7) ist: 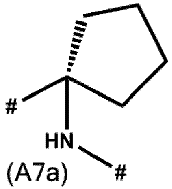 (A8) ist: 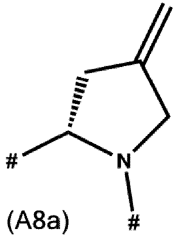 (A10) ist: 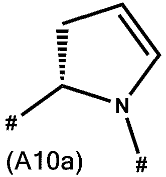 (A11) ist: 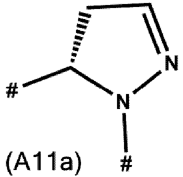 (A14) ist: 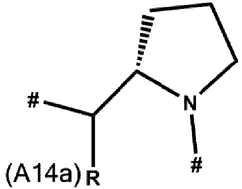 (A15) ist: 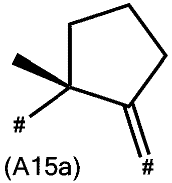 (A16) ist: 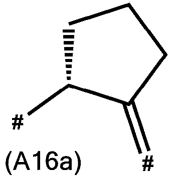 und (A17) ist:

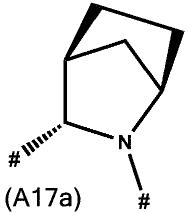

3. Das pharmazeutische Kombinationsprodukt nach Anspruch 1, wobei das antivirale Benzimidazolderivat der ***Formel I*** das antivirale Benzimidazolderivat der ***Formel Ia*:** 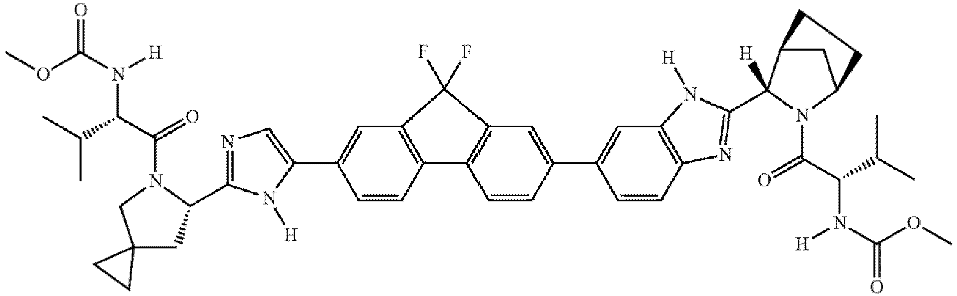
Methyl *N*-[(2*S*)-1-[(6*S*)-6-[5-[9,9-difluor-7-[2-[(1*R*,3*S*,4*S*)-2-[(2*S*)-2-(methoxycarbonylamino)-3-methylbutanoyl]-2-azabicyclo[2.2.1]heptan-3-yl]-3*H*-benzimidazol-5-yl]fluoren-2-yl]-1*H*-imidazol-2-yl]-5-azaspiro[2.4]heptan-5-yl]-3-methyl-1-oxobutan-2-yl]carbamat
oder ein pharmazeutisch akzeptables Salz davon ist.

4. Das pharmazeutische Kombinationsprodukt nach einem der Ansprüche 1 bis 3, wobei das Protoparvovirus H-1PV ist.

5. Das pharmazeutische Kombinationsprodukt nach einem der Ansprüche 1 bis 4, umfassend (a) das Protoparvovirus und (b) das antivirale Benzimidazolderivat als separate Einheiten.

6. Das pharmazeutische Kombinationsprodukt nach einem der Ansprüche 1 bis 4, umfassend (a) das Protoparvovirus und (b) das antivirale Benzimidazolderivat in einer einzigen Zusammensetzung.

7. Das pharmazeutische Kombinationsprodukt nach einem der Ansprüche 1 bis 6 zur Verwendung in der medizinischen Behandlung eines Patienten.

8. Das pharmazeutische Kombinationsprodukt zur Verwendung nach Anspruch 7, wobei die medizinische Behandlung eine Krebsbehandlung ist.

9. Das pharmazeutische Kombinationsprodukt zur Verwendung nach Anspruch 7 oder 8, wobei die Behandlung die Behandlung von soliden Tumoren oder krebsinitiierenden Vorläuferzellen ist.

10. Das pharmazeutische Kombinationsprodukt zur Verwendung nach Anspruch 8, wobei die Krebsbehandlung die Behandlung von Hirnkrebs, Bauchspeicheldrüsenkrebs, Nierenkrebs, Lungenkrebs oder Eierstockkrebs ist.

11. Das pharmazeutische Kombinationsprodukt zur Verwendung nach einem der Ansprüche 7 bis 10, wobei die Behandlung die Suszeptibilität eines Krebses für die Onkotoxizität des Protoparvovirus im Vergleich zur Suszeptibilität desselben Krebses für eine äquivalente Dosis des Protoparvovirus allein erhöht.

12. Das pharmazeutische Kombinationsprodukt zur Verwendung nach einem der Ansprüche 7 bis 11, wobei die Behandlung eine Behandlung eines Krebses ist, der resistent oder refraktär gegenüber der Onkotoxizität des Protoparvovirus ist.

13. Das pharmazeutische Kombinationsprodukt zur Verwendung nach einem der Ansprüche 7 bis 12, wobei die Behandlung umfasst: intratumorale, systemische oder intranasale Verabreichung (a) des Protoparvovirus; und intratumorale, systemische oder orale Verabreichung (b) des antiviralen Benzimidazolderivats.

14. Das pharmazeutische Kombinationsprodukt zur Verwendung nach Anspruch 13, wobei die Behandlung umfasst: intratumorale Verabreichung (a) des Protoparvovirus; und orale Verabreichung (b) des antiviralen Benzimidazolderivats.

15. Das pharmazeutische Kombinationsprodukt zur Verwendung nach einem der Ansprüche 7 bis 14, wobei die Behandlung die gleichzeitige oder sequentielle Verabreichung (a) des Protoparvovirus und (b) des antiviralen Benzimidazolderivats an den Patienten umfasst.

## Revendications

1. Produit d'association pharmaceutique comprenant :
(a) un protoparvovirus, ledit protoparvovirus étant H-1 (H-1PV) ou un protoparvovirus de rongeur apparenté choisi parmi Lulll, le microvirus de la souris (MMV), le protoparvovirus de la souris (MPV), le microvirus du rat (RMV), le protoparvovirus du rat (RPV) ou le virus du rat (RV) ; et
(b) un dérivé de benzimidazole antiviral de formule I : 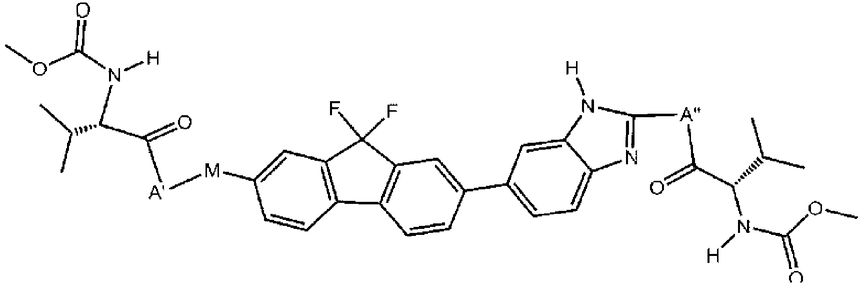
ou un sel pharmaceutiquement acceptable de celui-ci,
dans lequel, dans le dérivé de benzimidazole antiviral de formule I :
M est un cycle hétéroaryle de 5 chaînons ; et
A' et A" sont chacun indépendamment choisis parmi (A1) à (A17) : 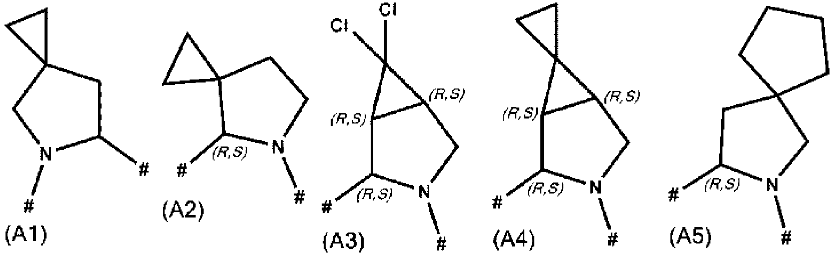 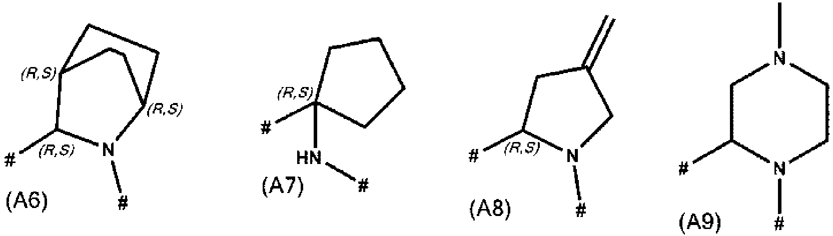 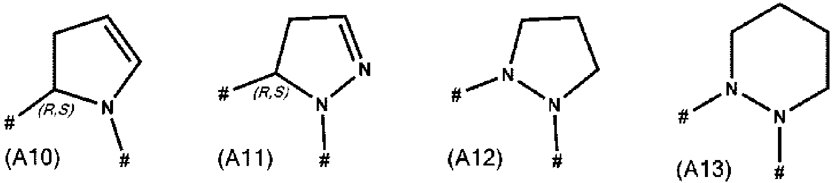 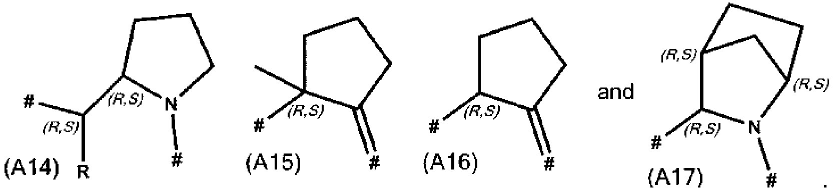
où R est hydrogène ou méthyle.

2. Produit d'association pharmaceutique selon la revendication 1, dans lequel (A2) est : 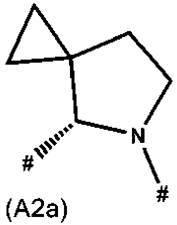 (A3) est : 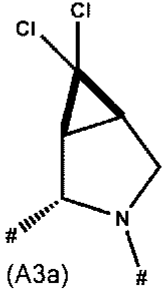 (A4) est : 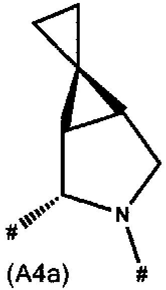 (A5) est : 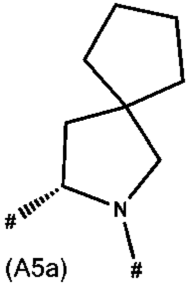 (A6) est : 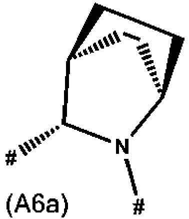 (A7) est : 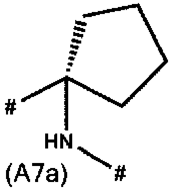 (A8) est : 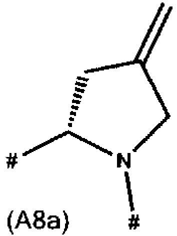 (A10) est: 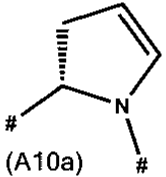 (A11) est: 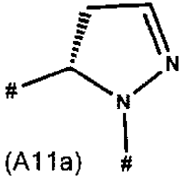 (A14) est : 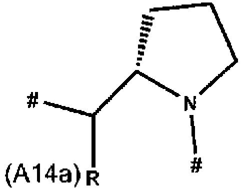 (A15) est 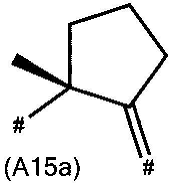 (A16) est : 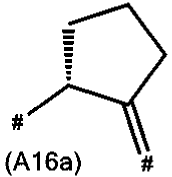 et (A17) est :

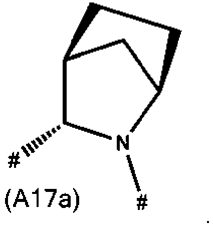

3. Produit d'association pharmaceutique selon la revendication 1, dans lequel le dérivé de benzimidazole antiviral de formule I est le dérivé de benzimidazole antiviral de formule la : 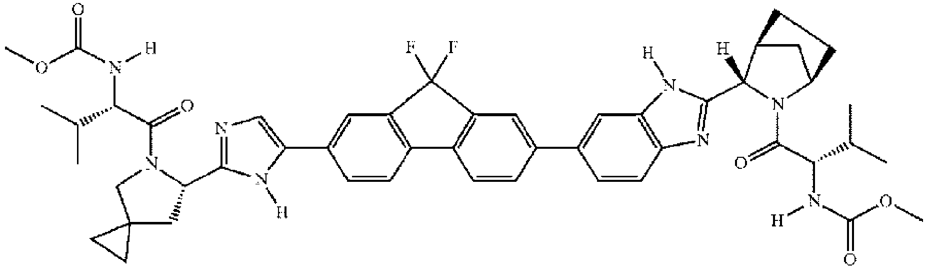
*N*-[(2*S*)-1-[(6*S*)-6-[5-[9,9-difluoro-7-[2-[(1*R*,3*S*,4*S*)-2-[(2*S*)-2-(méthoxycarbonylamino)-3-méthylbutanoyl]-2-azabicyclo[2.2.1]heptan-3-yl]-3*H*-benzimidazol-5-yl]fluorén-2-yl]-1*H-*imidazol-2-yl]-5-azaspiro[2.4]heptan-5-yl]-3-méthyl-1-oxobutan-2-yl]carbamate de méthyle,
ou un sel pharmaceutiquement de celui-ci.

4. Produit d'association pharmaceutique selon l'une quelconque des revendications 1 à 3, dans lequel le protoparvovirus est H-1 PV.

5. Produit d'association pharmaceutique selon l'une quelconque des revendications 1 à 4, comprenant (a) le protoparvovirus et (b) le dérivé de benzimidazole antiviral en tant qu'entités séparées.

6. Produit d'association pharmaceutique selon l'une quelconque des revendications 1 à 4, comprenant (a) le protoparvovirus et (b) le dérivé de benzimidazole antiviral dans une composition unique.

7. Produit d'association pharmaceutique selon l'une quelconque des revendications 1 à 6 pour utilisation dans le traitement médical d'un patient.

8. Produit d'association pharmaceutique pour utilisation selon la revendication 7, ledit traitement médical étant un traitement contre le cancer.

9. Produit d'association pharmaceutique pour utilisation selon la revendication 7 ou 8, ledit traitement étant un traitement de tumeurs solides ou de cellules précurseurs initiatrices de cancer.

10. Produit d'association pharmaceutique à utiliser selon la revendication 8, ledit traitement contre le cancer étant un traitement du cancer du cerveau, du cancer du pancréas, du cancer du rein, du cancer du poumon ou du cancer de l'ovaire.

11. Produit d'association pharmaceutique pour utilisation selon l'une quelconque des revendications 7 à 10, ledit traitement augmentant la susceptibilité d'un cancer à l'oncotoxicité du protoparvovirus par rapport à la susceptibilité dudit cancer à une dose équivalente de protoparvovirus seul.

12. Produit d'association pharmaceutique pour utilisation selon l'une quelconque des revendications 7 à 11, ledit traitement étant un traitement d'un cancer résistant ou réfractaire à l'oncotoxicité du protoparvovirus.

13. Produit d'association pharmaceutique pour utilisation selon l'une quelconque des revendications 7 à 12, ledit traitement comprenant : l'administration intratumorale, systémique ou intranasale (a) du protoparvovirus ; et l'administration intratumorale, systémique ou orale (b) du dérivé de benzimidazole antiviral.

14. Produit d'association pharmaceutique pour utilisation selon la revendication 13, ledit traitement comprenant : l'administration intratumorale (a) du protoparvovirus ; et l'administration orale (b) du dérivé de benzimidazole antiviral.

15. Produit d'association pharmaceutique pour utilisation selon l'une quelconque des revendications 7 à 14, ledit traitement comprenant l'administration (a) du protoparvovirus et (b) du dérivé de benzimidazole antiviral audit patient simultanément ou séquentiellement.
